# EUROPEAN PATENT SPECIFICATION

(11) **EP 3 714 797 B1**
(45) Date of publication and mention of the grant of the patent: **20.09.2023**
(21) Application number: 18897733.4
(22) Date of filing: 16.10.2018
(51) Int. Cl.: G01N 21/78, A61B 10/00, G01N 33/53, G01N 33/76, G01N 21/84, G01N 33/558, G01N 21/77, G01N 21/76

(54) **DIAGNOSTIC DEVICE AND DIAGNOSTIC METHOD**
DIAGNOSEVORRICHTUNG UND DIAGNOSEVERFAHREN
DISPOSITIF DE DIAGNOSTIC ET PROCÉDÉ DE DIAGNOSTIC

(30) Priority: 28.12.2017 KR 20170182705
(43) Date of publication of application: 30.09.2020
(73) Proprietor: Sugentech, Inc., Daejeon 34025 (KR)
(72) Inventor: YOO, Seung Bum, Daejeon 35263 (KR); LEE, Ri Mi, Daejeon 34023 (KR); KIM, Eun Kyung, Daejeon 34119 (KR); SOHN, Mi Jin, Daejeon 34076 (KR)
(74) Representative: Manitz Finsterwald Patent- und Rechtsanwaltspartnerschaft mbB
(86) International application number: PCT/KR2018/012147
(87) International publication number: WO 2019/132199

(56) References cited:
- WO-A1-2016/081453
- KR-A- 20010 023 529
- KR-A- 20090 008 175
- KR-A- 20090 008 175
- KR-A- 20160 108 695
- KR-B1- 101 068 612
- US-A1- 2015 094 227

## Description

### [Technical Field]

The present disclosure relates to a diagnostic device, a diagnostic device set and a diagnostic method.

### [Background Art]

Female ovulation is a phenomenon in which an egg matures and is released from an ovary into a fallopian tube, and is one step of a menstrual cycle. Women's menses occur periodically due to balanced action of various sex hormones, usually once a month (28 days). One egg matures and is released from the ovary. At this time, the woman's placenta creates an environment suitable for implantation of a fertilized egg thereto when the egg is fertilized with a sperm. In this connection, an endometrium is swollen. When the egg is not fertilized or the fertilized egg is not implanted, the endometrium collapses and the menstruation begins.

Ovulation is caused by various sex hormones such as follicle stimulating hormone (FSH), LH (Luteinizing Hormone), estrogen, progesterone, etc. When the hormones are released, the egg matures and ovulates. In preparation for pregnancy, the endometrium swells. When she is not pregnant, the endometrium collapses and the menstruation begins.

Female hormones have a variety of actions related to the menstrual cycle. When there are problems with female hormone generation or concentration, various symptoms such as premenstrual syndrome (PMS), premenstrual dysphoric disorder (PMDD), ovulation disorder, and ectopic pregnancy may occur. Therefore, the female hormones may be analyzed to diagnose various female diseases and health state.

U.S. Pat. No. 7,326,578 discloses a method for diagnosing menopause by analyzing a concentration of FSH via urine sample analysis. However, the patent has limitations in comprehensively determining women's health because a state which is diagnosed via analysis of a specific hormone is limited.
US 2015/094227 A1 relates to a test device to detect pregnancy in a human female subject, the test device comprising: an assay means to measure the absolute or relative amount of hCG in a sample from the subject; an assay means to measure the absolute or relative amount of FSH in a sample from the subject; and an assay means to measure the absolute or relative amount of one or more progesterone metabolites in a sample from the subject. KR 2009 0008175 A relates to a method for measuring the fertility of a mammal comprising obtaining a body fluid sample from a mammalian female subject; contacting the sample with a solid phase capture element comprising a first binding agent capable of binding an estrogen metabolite and a second binding agent capable of binding a progesterone metabolite; measuring secretion rates of the estrogen metabolite and the progesterone metabolite; and measuring the ovulation cycle state of the female subject based on the relative secretion rates of the estrogen metabolite and the progesterone metabolite.

### [Disclosure]

### [Technical Problem]

The present disclosure is intended to solve the problems of the prior art as described above, and aims to provide a diagnostic device, a diagnostic device set, and a diagnostic method.

However, the technical purpose to be achieved by the embodiments of the present disclosure is not limited to the technical purposes as described above. Other technical purposes may exist.

### [Technical Solution]

A first aspect of the present disclosure provides a diagnostic device for diagnosing a state of a subject based on a ratio or a difference between a color intensity of a first test portion and a color intensity of a second test portion in a reaction unit receiving a sample therein in accordance with claim 1.

In one implementation embodiment of the present disclosure, the diagnosis unit is configured to output normal information or abnormal information as information about the pregnancy related state, based on the first ratio and the second ratio. However, the present disclosure is limited thereto.

In one implementation embodiment of the present disclosure, the diagnosis unit is configured to: output the normal information when the first ratio is greater than the second ratio; or output the abnormal information when the first ratio is smaller than the second ratio. However, the present disclosure is limited thereto.

In one implementation embodiment of the present disclosure, the diagnosis unit is configured to: output the normal information when a value of the ratio gradually decreases from the first ratio to the second ratio as a time elapses from the first time-point to the second time-point; or output the abnormal information when a value of the ratio gradually increases from the first ratio to the second ratio as a time elapses from the first time-point to the second time-point. However, the present disclosure is limited thereto.

In one implementation embodiment of the present disclosure, the normal information indicates normal pregnancy or normal pregnancy maintenance, and the abnormal information indicates abnormal pregnancy or miscarriage. However, the present disclosure is limited thereto.

In one implementation embodiment of the present disclosure, the reaction unit includes: a sample pad receiving the sample therein; a conjugate pad; a reaction membrane having two or more test portions; and a wicking pad. However, the present disclosure is limited thereto.

In one implementation embodiment of the present disclosure, each of the first test portion and/or the second test portion has an individual antigen or antibody coating. However, the present disclosure is limited thereto.

In one implementation embodiment of the present disclosure, the reaction unit further include a control portion. However, the present disclosure is limited thereto.

In one implementation embodiment of the present disclosure, the diagnosis unit is configured to: output the normal information when the first ratio is smaller than the second ratio; or output the abnormal information when the first ratio is greater than the second ratio. However, the present disclosure is limited thereto.

In one implementation embodiment of the present disclosure, the diagnosis unit is configured to: output the normal information when a value of the ratio gradually increases from the first ratio to the second ratio as a time elapses from the first time-point to the second time-point; or output the abnormal information when a value of the ratio gradually decreases from the first ratio to the second ratio as a time elapses from the first time-point to the second time-point. However, the present disclosure is limited thereto.

In one implementation embodiment of the present disclosure, the normal information indicates that the subject is able to be pregnant, wherein the abnormal information indicates that the subject is not able to be pregnant or is in a menopause state. However, the present disclosure is limited thereto.

In one implementation embodiment of the present disclosure, the diagnosis unit is configured to: when the first ratio is a minimum value among a plurality of ratios including the first ratio and the second ratio, output time-point information corresponding to the first time-point as information about the pregnancy related state. However, the present disclosure is limited thereto.

In one implementation embodiment of the present disclosure, the time-point information is time-point information about ovulation. However, the present disclosure is limited thereto.

In one implementation embodiment of the present disclosure, the diagnosis unit is configured to: compare a plurality of ratios including the first ratio and the second ratio with each other; and when ratios below a threshold ratio last for a predetermined period, output the abnormal information. However, the present disclosure is limited thereto.

In one implementation embodiment of the present disclosure, the sample may include a substance selected from the group consisting of urine, blood, plasma, serum, lymph fluid, bone marrow fluid, saliva, milk, ocular fluid, semen, brain extract, spinal fluid, joint fluid, thymus fluid, ascites, amniotic fluid, cell tissue fluid, buffer solution, tap water, waste water, sewage, groundwater and combinations thereof. However, the present disclosure is limited thereto.

In one implementation embodiment of the present disclosure, the analyte includes a hormone selected from the group consisting of follicle stimulating hormone, luteinizing hormone, estrogen, progesterone, estradiol, human chorionic gonadotropin, and combinations thereof. However, the present disclosure is limited thereto.

In one implementation embodiment of the present disclosure, the reaction membrane may include a material selected from the group consisting of nitrocellulose, cellulose, polyethylene terephthalate (PET), polyethersulfone (PES), glass fiber, nylon and combinations thereof. However, the present disclosure is limited thereto.

In one implementation embodiment of the present disclosure, the state of the subject is diagnosed using a method selected from the group consisting of immunochromatography, ELISA, radioimmunoassay, enzyme immunoassay, particle aggregation assay, chemiluminescence immunoassay, real-time polymerase reaction, flow cytometry, immunosensor, radial immunodiffusion, rocket immunoelectrophoresis, two-dimensional electrophoretic analysis, liquid chromatography-mass spectrometry (LC-MS), Western blot, and combinations thereof. However, the present disclosure is limited thereto.

In one implementation embodiment of the present disclosure, the diagnosis unit includes a light emitting unit and a light receiving unit. However, the present disclosure is limited thereto.

In one implementation embodiment of the present disclosure, the light receiving unit measures the color intensity based on an amount of light reflected from the reaction unit when the diagnosis unit irradiates light on the reaction unit. However, the present disclosure is limited thereto.

In one implementation embodiment of the present disclosure, the device further includes a wireless communication module. However, the present disclosure is limited thereto.

A second aspect of the present disclosure provides a diagnostic device set including the diagnostic device according to claim 1 and an analysis reader into which the diagnostic device is inserted.

A third aspect of the present disclosure provides a diagnostic method in accordance with claim 23.

The above-mentioned implementations are merely exemplary and should not be construed as intended to limit the present disclosure. The invention is limited only by the scope of the appended claims. In addition to the exemplary embodiments as described above, additional embodiments may be present in the drawings and detailed description of the disclosure.

### [Advantageous Effects]

According to the present disclosure, the diagnostic device according to the present disclosure may analyze two or more analytes simultaneously, thereby enabling more accurate diagnosis of the disease. In particular, analyzing the ratio or difference between two or more analytes may allow predicting polycystic ovary syndrome, which may not be diagnosed in the prior art in which diagnosis is based on a cut-off. Moreover, two or more analytes may be analyzed simultaneously to analyze patterns of analytes such as various hormone to more accurately predict and determine diseases.

The diagnostic device set according to the present disclosure includes the analysis reader. Thus, self-diagnosis may be realized by the analysis reader analyzing the color intensities of the test portion and/or the control portion of the diagnostic device and displaying the results. Moreover, since the diagnostic device may be connected to a wireless communication module. Thus, the state of the subject may be checked in real time in conjunction with an electronic device such as a smartphone. Furthermore, the diagnostic device where test portions are configured based on analytes varying depending on the disease or state to be diagnosed may be used in various fields such as diagnosis and prediction of disease, health care, paternity test, constitution analysis, fermentation engineering, biotechnology, food safety test, environmental analysis, cosmetic analysis and compound analysis.

### [Description of Drawings]

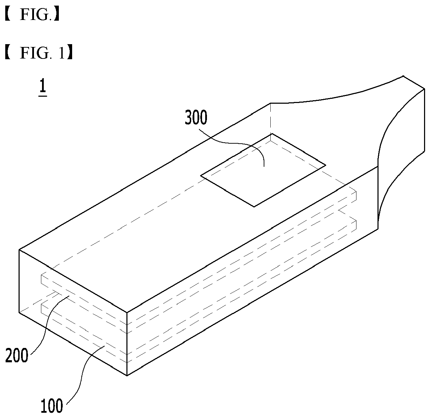

FIG. 1 is a schematic diagram of a diagnostic device according to one implementation embodiment of the present disclosure.

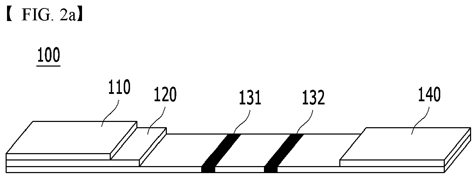

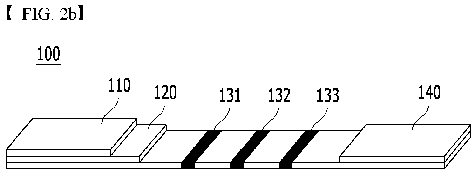

FIG. 2A and FIG. 2B are schematic diagrams of a reaction unit according to one implementation embodiment of the present disclosure.

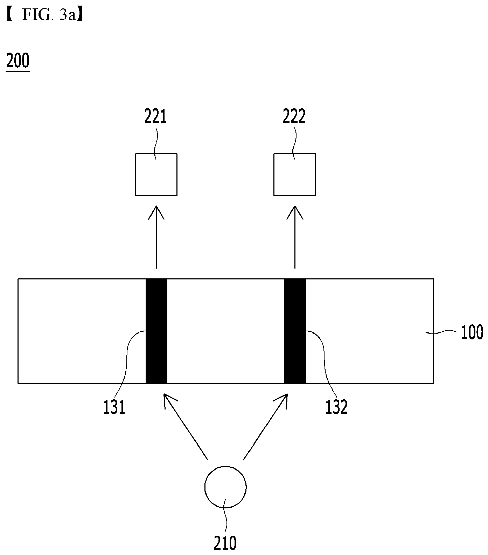

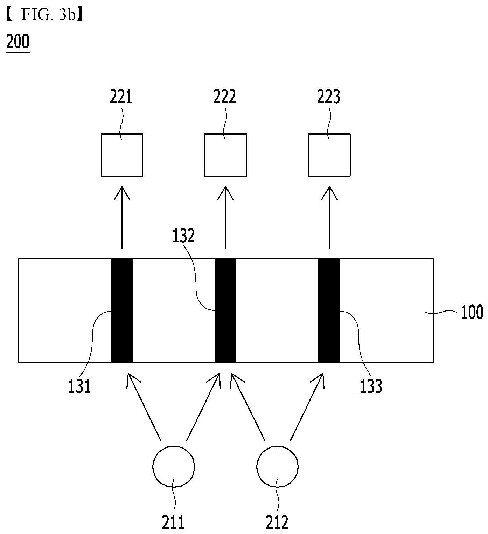

FIG. 3A and FIG. 3B are schematic diagrams of a diagnosis unit according to one implementation embodiment of the present disclosure.

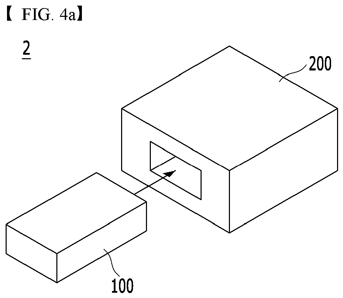

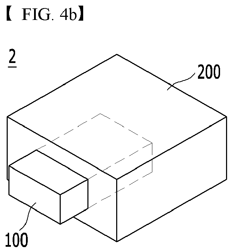

FIG. 4A and FIG. 4B are schematic diagrams of a diagnostic device set according to one implementation embodiment of the present disclosure.

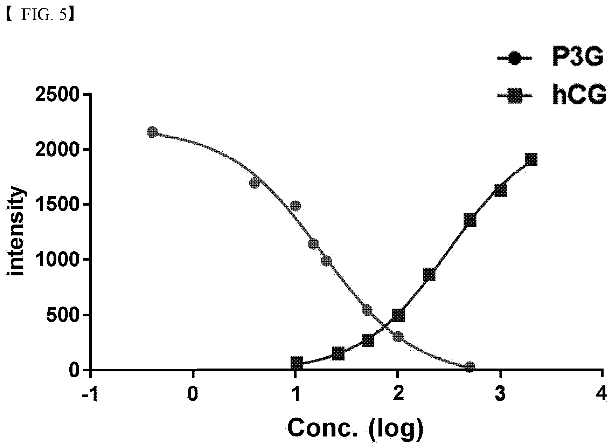

FIG. 5 is a graph showing change in a color intensity according to a hormone concentration in a pregnancy check diagnostic device according to one embodiment of the present disclosure.

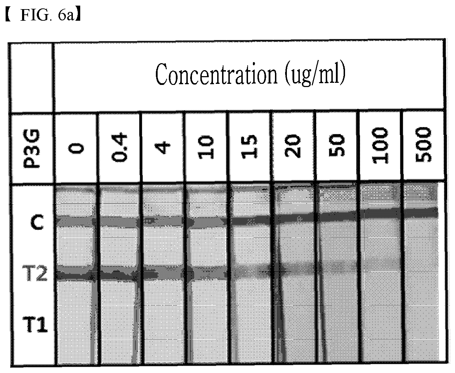

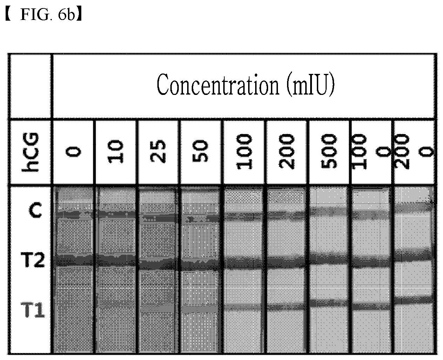

FIG. 6A is a real color picture according to a concentration of progesterone metabolites in a pregnancy check diagnostic device according to one embodiment of the present disclosure. FIG. 6B is a real color picture according to a concentration of chorionic gonadotropin in the pregnancy check diagnostic device according to one embodiment of the present disclosure.

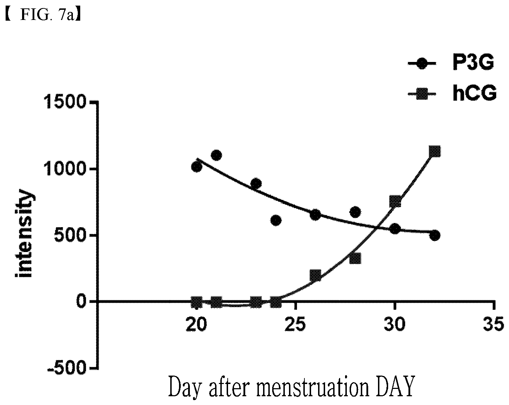

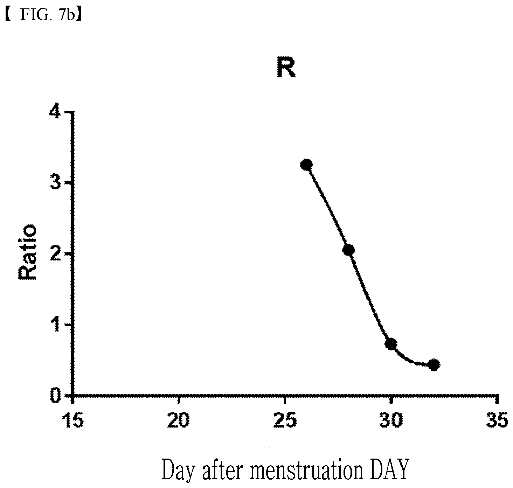

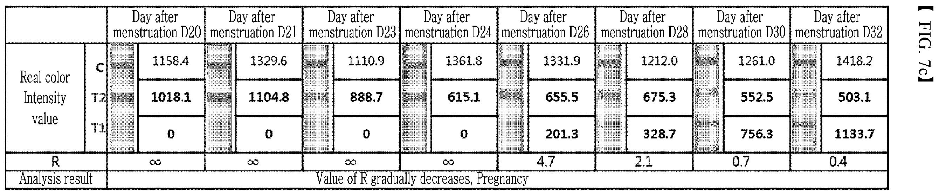

FIG. 7A is a graph showing a color intensity due to hormone over time after menstruation in a pregnancy check diagnostic device according to one embodiment of the present disclosure. FIG. 7B is a graph showing a ratio of hormone in FIG. 7A. FIG. 7C shows real color pictures and results according to a concentration of hormones in the pregnancy check diagnostic device according to one embodiment of the present disclosure.

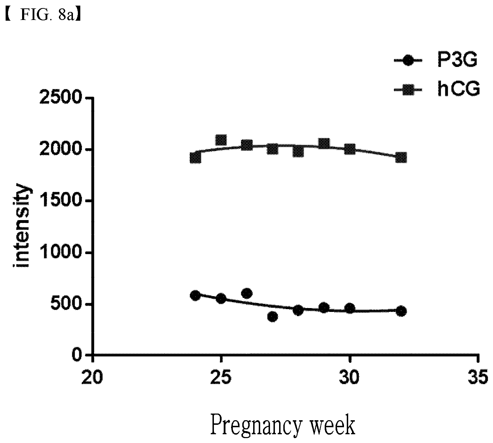

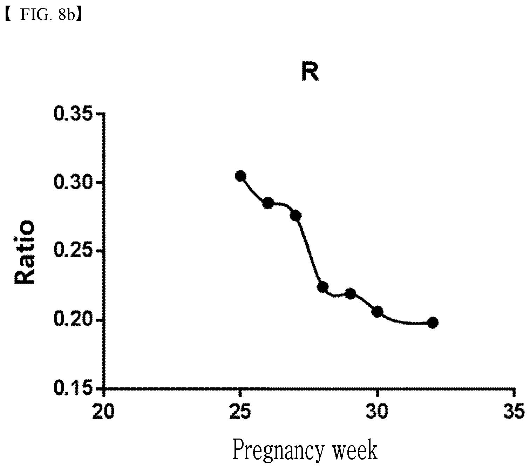

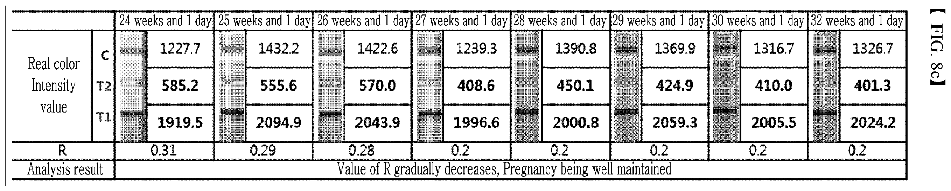

FIG. 8A is a graph showing a color intensity due to hormone over time after menstruation in a pregnancy check diagnostic device according to one embodiment of the present disclosure. FIG. 8B is a graph showing a ratio of hormone in FIG. 8A. FIG. 8C shows real color pictures and results according to a hormone concentration in the pregnancy check diagnostic device according to one embodiment of the present disclosure.

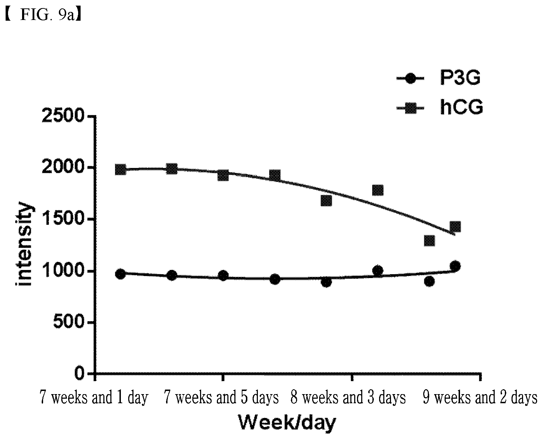

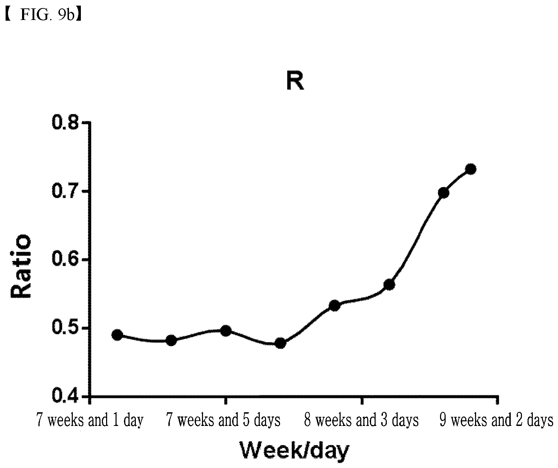

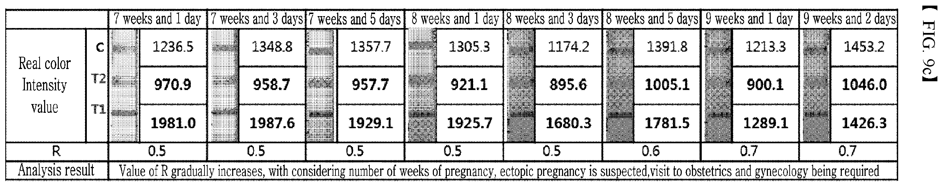

FIG. 9A is a graph showing a color intensity due to hormone over time after menstruation in a pregnancy check diagnostic device according to one embodiment of the present disclosure. FIG. 9B is a graph showing a ratio of hormone in FIG. 9A. FIG. 9C shows a real color picture and results according to a concentration of hormones in the pregnancy check diagnostic device according to one embodiment of the present disclosure.

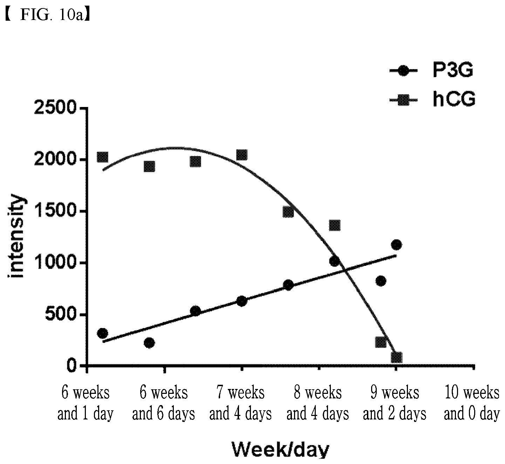

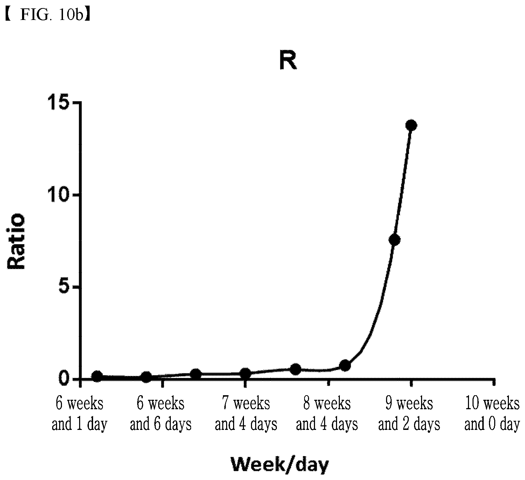

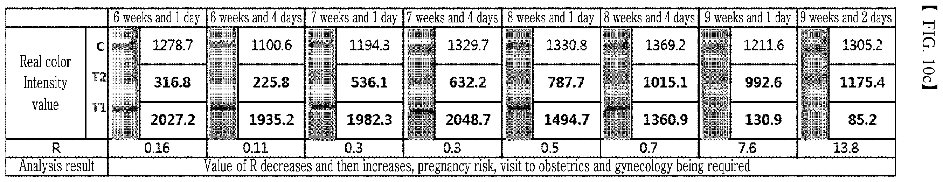

FIG. 10A is a graph showing a color intensity due to hormone over time after menstruation in a pregnancy check diagnostic device according to one embodiment of the present disclosure. FIG. 10B is a graph showing a ratio of hormone in FIG. 10A. FIG. 10C shows a real color picture and results according to a concentration of hormones in the pregnancy check diagnostic device according to one embodiment of the present disclosure.

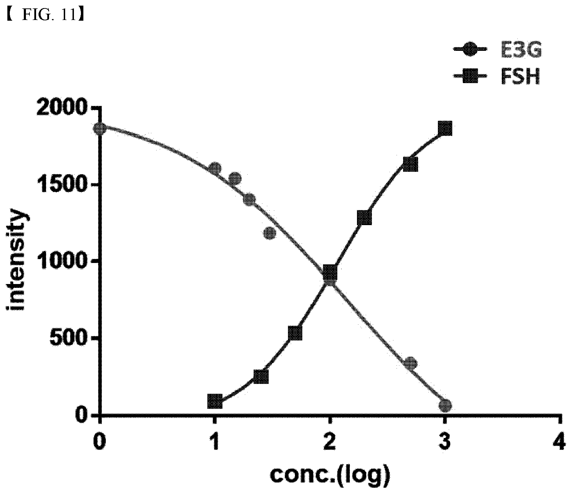

FIG. 11 is a graph showing change in a color intensity according to a hormone concentration in a diagnostic device for menopause check according to one embodiment of the present disclosure.

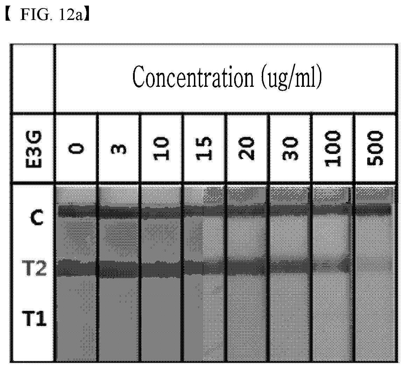

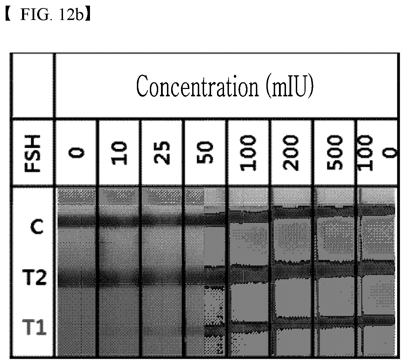

FIG. 12A is a real color picture according to a concentration of estrogen metabolites in a diagnostic device for menopause check according to one embodiment of the present disclosure. FIG. 12B is a real color picture according to a concentration of follicle stimulating hormone in the diagnostic device for menopause check according to one embodiment of the present disclosure.

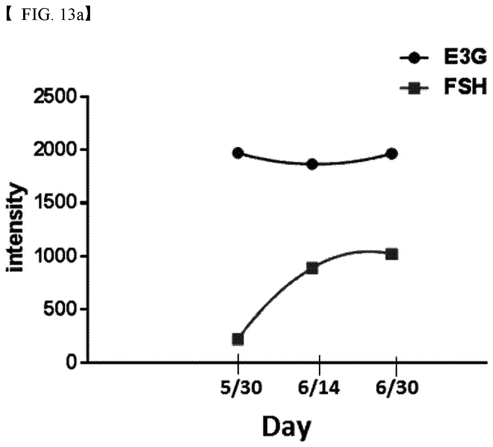

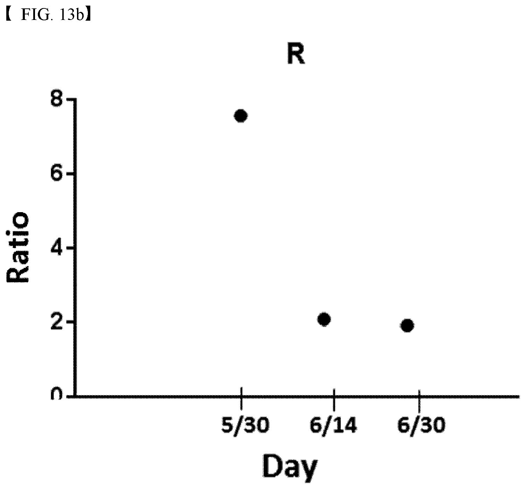

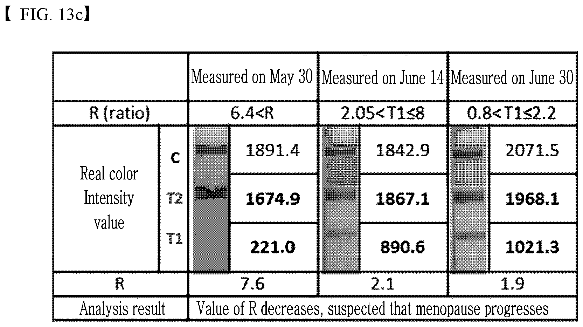

FIG. 13A is a graph showing a color intensity due to hormone over time in a diagnostic device for menopause check according to one embodiment of the present disclosure. FIG. 13B is a graph showing a ratio of hormone in FIG. 13A. FIG. 13C shows a real color picture and results according to a concentration of hormones in the diagnostic device for menopause check according to one embodiment of the present disclosure.

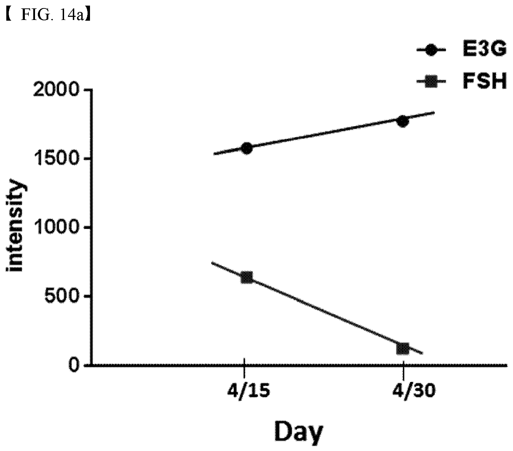

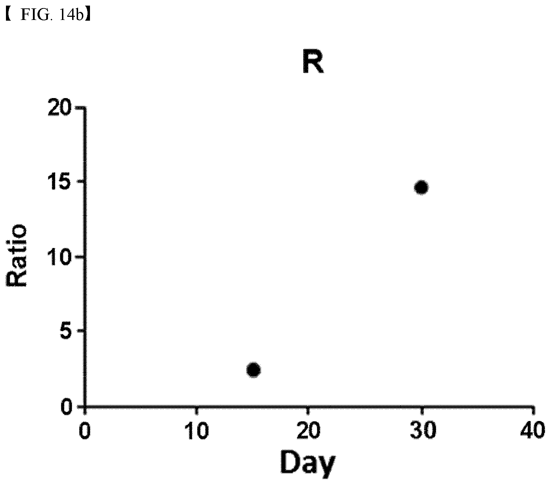

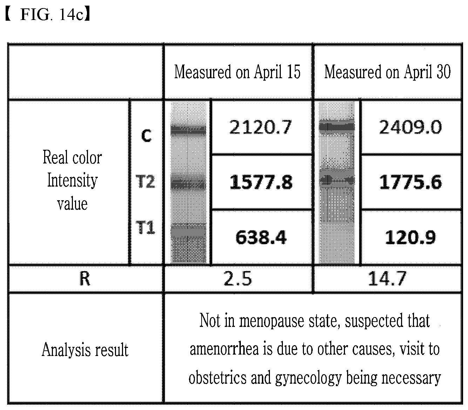

FIG. 14A is a graph showing a color intensity due to hormone over time in a diagnostic device for menopause check according to one embodiment of the present disclosure. FIG. 14B is a graph showing a ratio of hormone in FIG. 14A. FIG. 14C shows a real color picture and results according to a hormone concentration in the diagnostic device for menopause check according to one embodiment of the present disclosure.

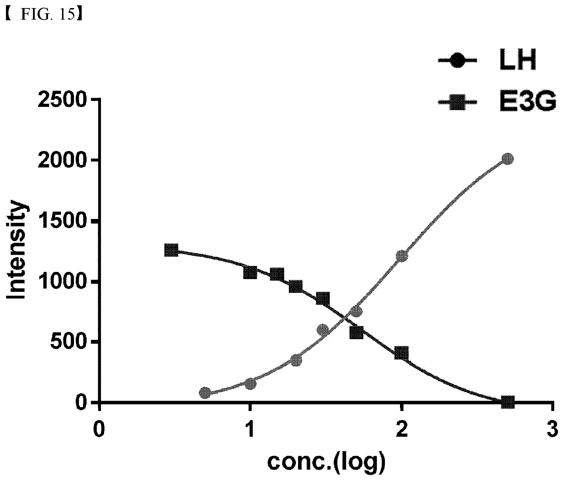

FIG. 15 is a graph showing change in a color intensity according to a hormone concentration in a diagnostic device for ovulation check according to one embodiment of the present disclosure.

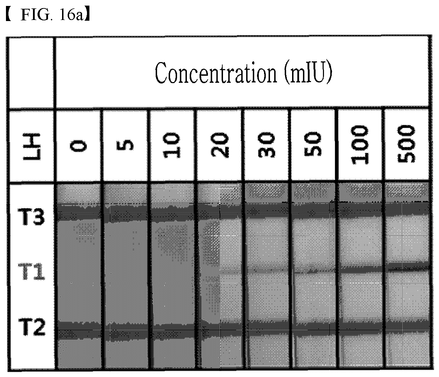

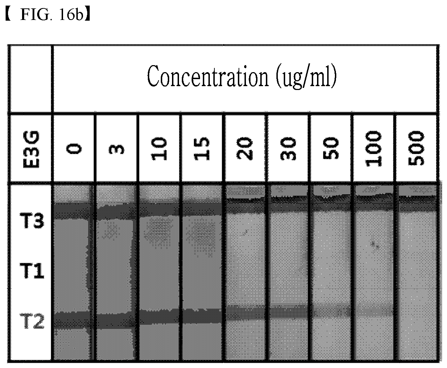

FIG. 16A is a real color picture according to a concentration of luteinizing hormone in a diagnostic device for ovulation check according to one embodiment of the present disclosure. FIG. 16B is a real color picture according to a concentration of estrogen metabolites in the diagnostic device for ovulation check according to one embodiment of the present disclosure.

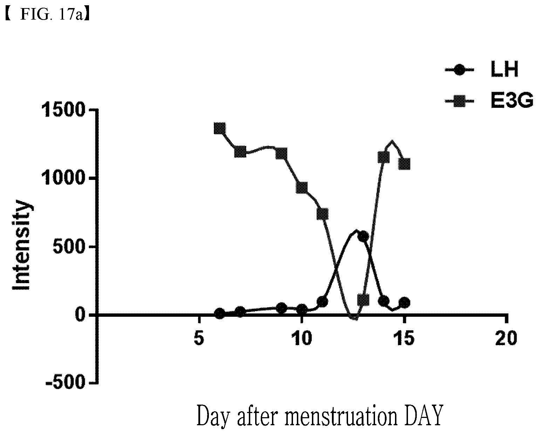

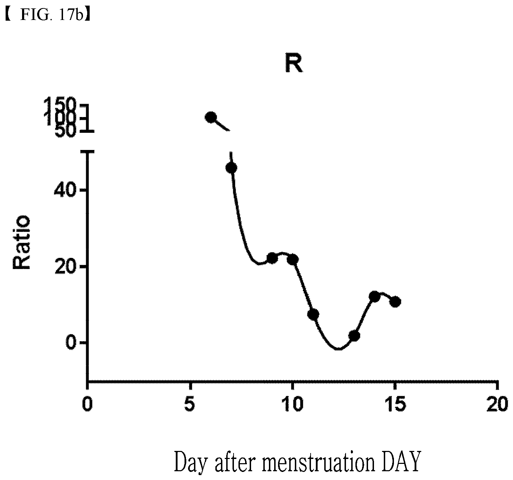

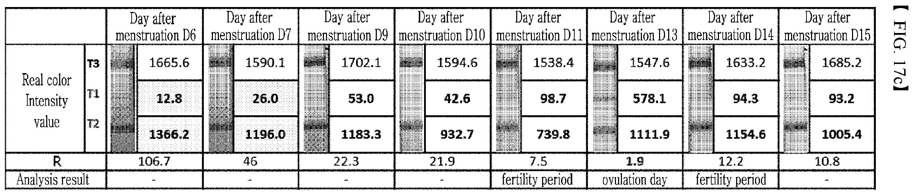

FIG. 17A is a graph showing a color intensity due to hormone over time after menstruation in a diagnostic device for ovulation check according to one embodiment of the present disclosure. FIG. 17B is a graph showing a ratio of hormone in FIG. 17A. FIG. 17C shows a real color picture and results according to a concentration of hormones in the diagnostic device for ovulation check according to one embodiment of the present disclosure.

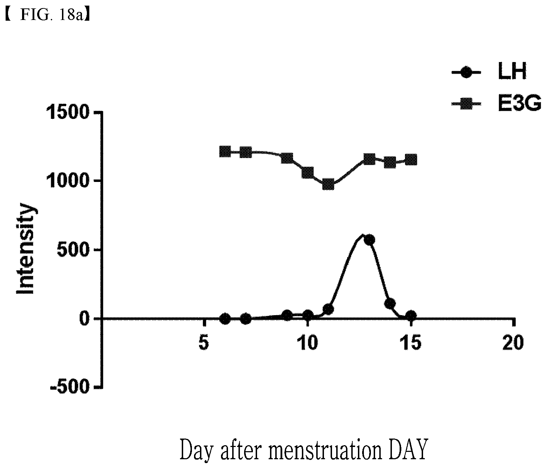

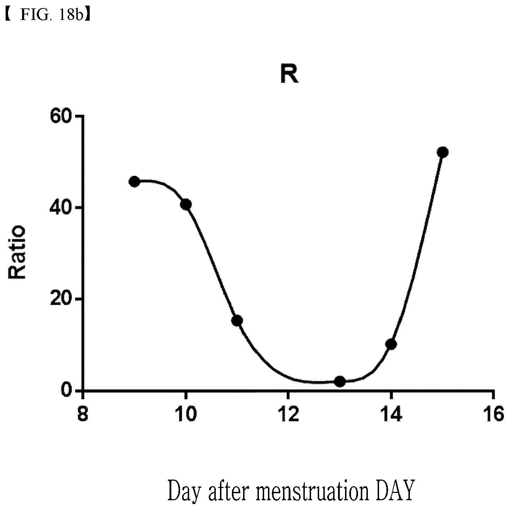

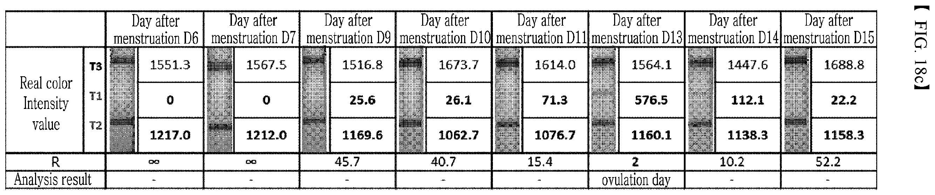

FIG. 18A is a graph showing a color intensity of hormones over time after menstruation in a diagnostic device for ovulation check according to one embodiment of the present disclosure. FIG. 18B is a graph showing a ratio of hormone in FIG. 18A. FIG. 18C shows a real color picture and results according to a concentration of hormone in the diagnostic device for ovulation check according to one embodiment of the present disclosure.

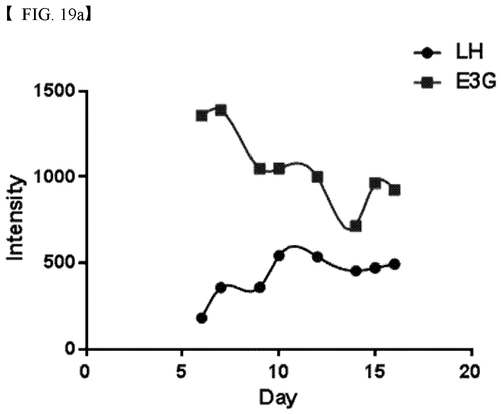

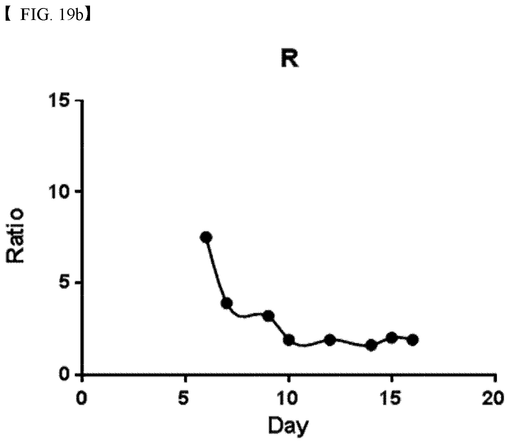

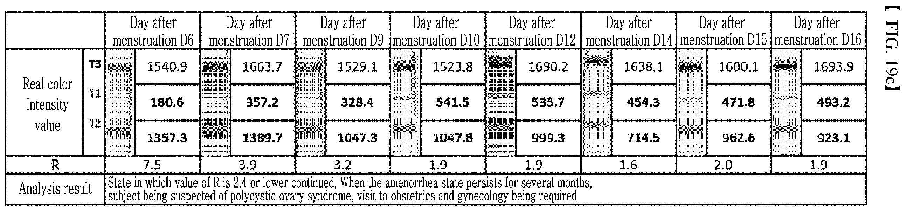

FIG. 19A is a graph showing a color intensity of hormones over time after menstruation in a diagnostic device for ovulation check according to one embodiment of the present disclosure. FIG. 19B is a graph showing a ratio of hormone in FIG. 19A. FIG. 19C shows a real color picture and results according to a concentration of hormones in the diagnostic device for ovulation check according to one embodiment of the present disclosure.

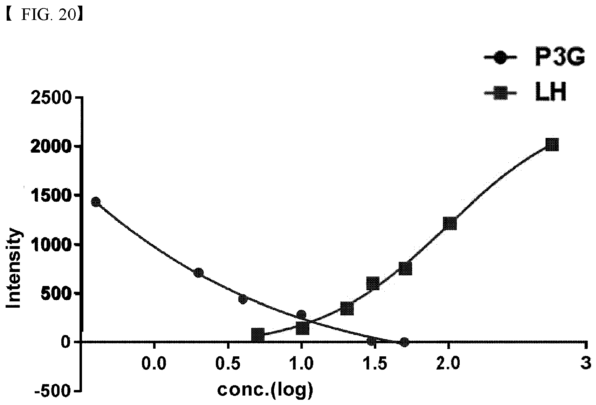

FIG. 20 is a graph showing change in a color intensity according to a hormone concentration in a diagnostic device for ovulation check according to one embodiment of the present disclosure.

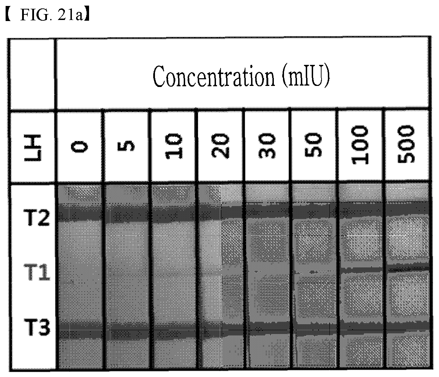

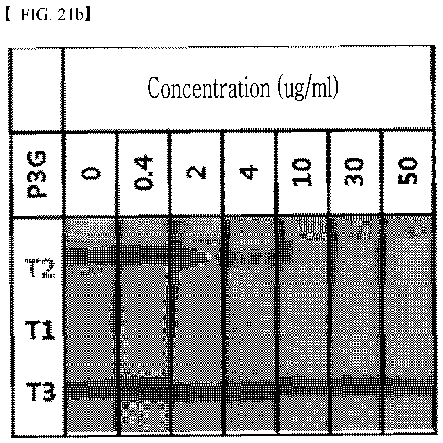

FIG. 21A is a real color picture according to a concentration of luteinizing hormone in the diagnostic device for ovulation check according to one embodiment of the present disclosure. FIG. 21B is a real color picture according to a concentration of progesterone metabolites in the diagnostic device for ovulation check according to one embodiment of the present disclosure.

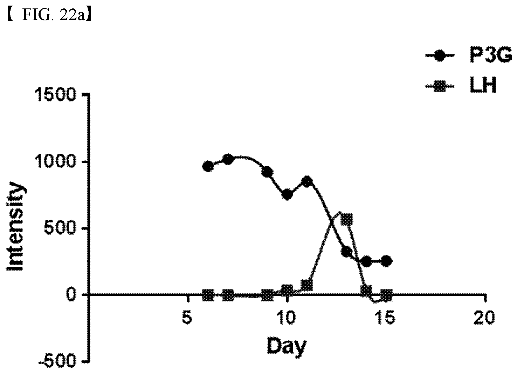

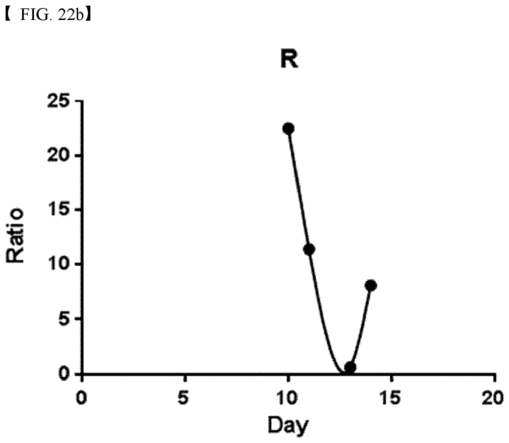

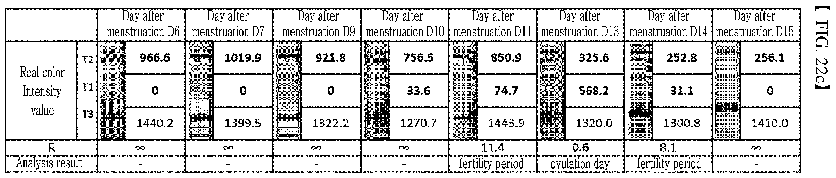

FIG. 22A is a graph showing a color intensity due to hormone over time after menstruation in a diagnostic device for ovulation check according to one embodiment of the present disclosure. FIG. 22B is a graph showing a ratio of hormone in FIG. 22A. FIG. 22C shows a real color picture and results according to a concentration of hormone in the diagnostic device for ovulation check according to one embodiment of the present disclosure.

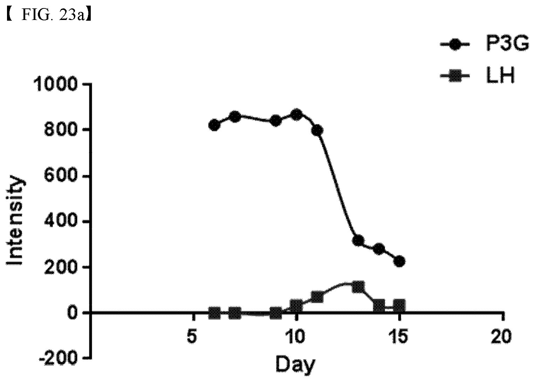

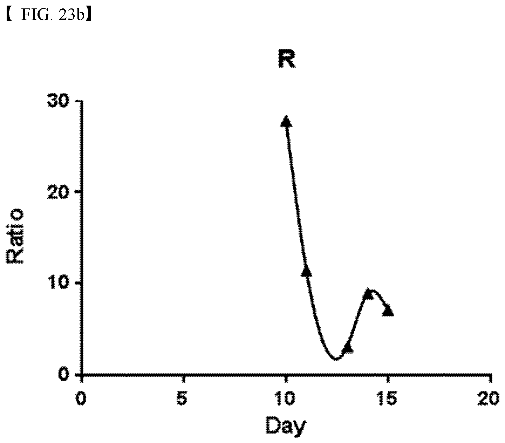

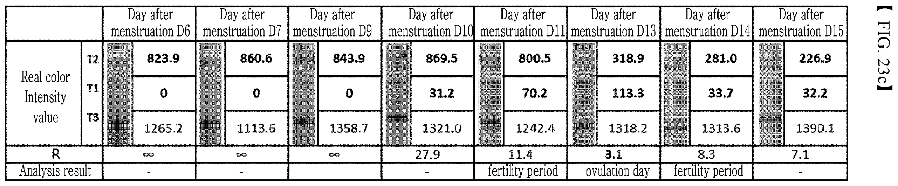

FIG. 23A is a graph showing a color intensity due to hormone over time after menstruation in a diagnostic device for ovulation check according to one embodiment of the present disclosure. FIG. 23B is a graph showing a ratio of hormone in FIG. 23A. FIG. 23C shows a real color picture and results according to a concentration of hormone in the diagnostic device for ovulation check according to one embodiment of the present disclosure.

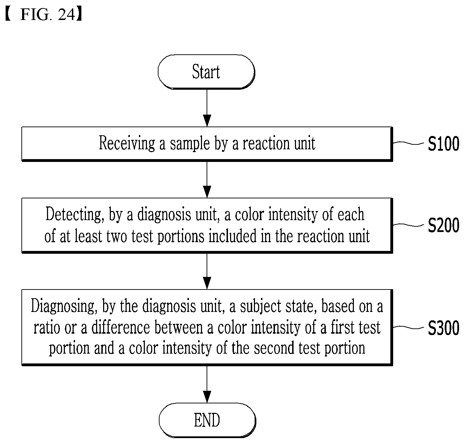

FIG. 24 is a flowchart of a diagnostic method according to one implementation embodiment of the present disclosure.

### [Description of Main Reference Numerals of Drawings]

1: Diagnostic device
2: Diagnostic device set
100: Reaction unit
110: Sample pad
120: Conjugate pad
130: Reaction membrane
131: First test portion
132: Second test portion
133: Control portion
140: Wicking pad
200: Diagnosis unit
210: Light emitting unit
211: Light emitting unit
212: Light emitting unit
221: Light receiving unit
222: Light receiving unit
223: Light receiving unit

### [Best Mode]

Hereinafter, an embodiment of the present disclosure will be described in detail with reference to the accompanying drawings so that those skilled in the art to which the present disclosure belongs may easily implement the disclosure.

However, the present disclosure may be implemented in many different forms and is not limited to the embodiments described herein. In order to clearly illustrate the present disclosure, parts not related to the description are omitted in the drawings. Similar reference numerals are used for similar parts throughout the specification.

It will be understood that when an element or layer is referred to as being "connected to", or "coupled to" another element or layer, it may be directly on, connected to, or coupled to the other element or layer, or one or more electrical-intervening elements or layers may be present.

In addition, it will also be understood that when a first element or layer is referred to as being present "on" or "beneath" a second element or layer, the first element may be disposed directly on or beneath the second element or may be disposed indirectly on or beneath the second element with a third element or layer being disposed between the first and second elements or layers.

It will be further understood that the terms "comprises", "comprising", "includes", and "including" when used in this specification, specify the presence of the stated features, integers, operations, elements, and/or components, but do not preclude the presence or addition of one or more other features, integers, operations, elements, components, and/or portions thereof.

As used herein, the terms "about", "substantially" about a specific value cover manufacturing and substance tolerances. The terms may prevent unscrupulous use by an infringer of a disclosure in which accurate or absolute values are set forth to aid in understanding of the present disclosure. Moreover, throughout the present specification, "step to" or "step of" does not mean "step for".

As used herein, the term "combinations thereof' included in a expression of Markush type means mixtures or combinations of at least one selected from the group consisting of components described in the expression of Markush type and means including one or more selected from the group consisting of the components.

As used herein, the term "and/or" includes any and all combinations of one or more of the associated listed items. Expression such as "at least one of" when preceding a list of elements may modify the entire list of elements and may not modify the individual elements of the list.

Hereinafter, a diagnostic device, a diagnostic device set, and a diagnostic method according to the present disclosure will be described in detail with reference to implementation embodiments and embodiments and drawings. However, the present disclosure is not limited to these implementation embodiments and embodiments and drawings.

A first aspect of the present disclosure provides a diagnostic device for diagnosing a state of a subject based on a ratio or a difference between a color intensity of a first test portion and a color intensity of a second test portion in a reaction unit receiving a sample therein, as defined with appended independent claim 1.

In one implementation embodiment of the present disclosure, the device includes, *inter alia* and as further defined with claim 1: the reaction unit receiving the sample therein and having two or more test portions; and a diagnosis unit for diagnosing the state of the subject based on the ratio or the difference between the color intensity of the first test portion and the color intensity of the second test portion. However, the present disclosure is limited thereto.

FIG. 1 is a schematic diagram of the diagnostic device according to one implementation embodiment of the present disclosure.

Specifically, a diagnostic device 1 according to one implementation embodiment of the present disclosure includes, *inter alia,* a reaction unit 100, a diagnosis unit 200 and a display 300. However, the present disclosure is limited thereto.

FIG. 2A and FIG. 2B are schematic diagrams of the reaction unit according to one implementation embodiment of the present disclosure.

Specifically, the reaction unit 100 according to one implementation embodiment of the present disclosure includes a sample pad 110, a conjugate pad 120, a reaction membrane 130 and a wicking pad 140. the reaction membrane 130 has two or more test portions. The subject state is diagnosed based on - *inter alia* - the color intensity of the first test portion 131 and the color intensity of the second test portion 132. However, the present disclosure is limited thereto.

Moreover, the reaction membrane 130 may further include a control portion 133. However, the present disclosure is limited thereto.

The display 300 may display the diagnostic information. However, the present disclosure is limited thereto.

The display 300 may include at least one of liquid crystal display (LCD), thin film transistor liquid crystal display (TFT LCD), organic light-emitting diode (OLED), flexible display, three-dimensional (3D) display, electronic-ink (e-ink) display. However, the present disclosure is limited thereto.

The diagnosis unit is configured to diagnose a pregnancy related state of the subject, based on the ratio or the difference between the color intensity of the first test portion and the color intensity of the second test portion, wherein the color intensity of the first test portion is higher as an analyte concentration increases, and the color intensity of the second test portion is lower as the analyte concentration increases. However, the present disclosure is limited thereto.

According to one implementation embodiment of the present disclosure, the reaction membrane may further include the control portion. However, the present disclosure is limited thereto.

The control portion refers to a line that is used to confirm whether the reaction occurs properly, but may contain a control substance. However, the present disclosure is limited thereto. In a control zone, reaction occurs regardless of presence or absence of analyte in the sample. Thus, when this reaction does not occur, the result of the test portion is determined as an error.

The state of the subject is diagnosed based on - *inter alia* - a ratio or a difference between the color intensity of the control portion and the color intensity displayed on the test portion. However, the present disclosure is limited thereto.

The control portion may be constructed in various ways. For example, when analyte is detected in a sandwich immunoassay scheme, a control zone may be constructed to have an antibody capable of binding to a label-target antibody, for example, a gold particle-target antibody complex.

In one implementation embodiment of the present disclosure, the reaction membrane may include a material selected from the group consisting of nitrocellulose, cellulose, polyethylene terephthalate (PET), polyethersulfone (PES), glass fiber, nylon and combinations thereof. However, the present disclosure is limited thereto.

In one implementation embodiment of the present disclosure, the reaction unit includes: a sample pad receiving the sample therein; a conjugate pad; a reaction membrane having two or more test portions; and a wicking pad. However, the present disclosure is limited thereto.

In one implementation embodiment of the present disclosure, the sample may include a substance selected from the group consisting of urine, blood, plasma, serum, lymph fluid, bone marrow fluid, saliva, milk, ocular fluid, semen, brain extract, spinal fluid, joint fluid, thymus fluid, ascites, amniotic fluid, cell tissue fluid, buffer solution, tap water, waste water, sewage, groundwater and combinations thereof. However, the present disclosure is limited thereto.

The sample pad may include a reaction substance that reacts with the analyte present in the sample. However, the present disclosure is limited thereto.

The reaction substance may be a capturing agent binding to the analyte. For example, the reaction substance may be selected from the group consisting of protein, gene, lipid, carbohydrate, vitamin or drug or a substance binding thereto, but is not limited thereto. The reaction substance may be selected from the group consisting of antibodies, receptor, streptavidin or avidin, aptamer, lectin, DNA, RNA, ligand, coenzyme, inorganic ion, enzyme cofactor, sugar, lipid, substrate and combinations thereof. However, the present disclosure is limited thereto.

According to one implementation embodiment of the present disclosure, the analyte may be a hormone selected from the group consisting of follicle stimulating hormone, luteinizing hormone, estrogen, progesterone, estradiol, human chorionic gonadotropin, and combinations thereof. However, the present disclosure is limited thereto.

The capturing agent may be immobilized in the test zone. The immobilization may be achieved via adsorption, hydrophobic interaction, hydrogen bonding, ionic bonding and/or covalent bonding. However, the present disclosure is limited thereto.

The reaction may be carried out using a method selected from the group consisting of immunochromatography, ELISA, radioimmunoassay, enzyme immunoassay, particle aggregation assay, chemiluminescence immunoassay, real-time polymerase reaction, flow cytometry, immunosensor, radial immunodiffusion, rocket immunoelectrophoresis, two-dimensional electrophoretic analysis, liquid chromatography-mass spectrometry (LC-MS), Western blot, and combinations thereof. However, the present disclosure is limited thereto.

When the reaction substance reacting with the analyte included in the test zone is a capturing agent, the diagnosis device may further include a detection agent. According to one implementation embodiment, in a rapid kit using immunochromatography, the detection agent may be included in the conjugate pad (conjugated pad).

The detection agent may be a target antibody, DNA, aptamer, streptavidin. However, the present disclosure is limited thereto. For example, when the capturing agent is a capturing antibody, the detection agent is a target antibody.

When the analyte is present in the sample, the detection agent may have a label binding thereto capable of generating an optical signal indicating the presence of the analyte.

The label includes various labels known in the art. For example, the label includes enzymes such as alkaline phosphatase, β-galactosidase, horseradish peroxidase, β-glucosidase and cytochrome P450, gold particles such as gold nanoparticles, silver particles such as silver nanoparticles, fluorescent substances such as fluorescein, FITC (fluoresein isothiocyanate), rhodamine 6G, rhodamine B, TAMRA (6-carboxy-tetramethyl-rhodamine), Cy-3, Cy-5, Texas Red, Alexa Fluor, DAPI (4, 6-diamidino-2-phenylindole), Coumarin, etc., fluorescent dyes, pigments such as gardenia pigment, eosin, phenol red, bromophenol blue, m-cresol purple and bromocresol purple, latex particles, chemiluminescent substances, and combinations thereof.

When the device includes the detection agent, the control portion may include a substance binding to the detection agent as a control substance. However, the present disclosure is limited thereto. For example, when the detection agent is a target antibody, the control substance is an antibody capable of binding to the target antibody.

A portion excluding the test portion and the control portion on the reaction membrane is referred to as a background zone. The background zone has a background environment around the test portion and the control portion. The background zone has the same environment as an environment having a substance except for the substances used for the reaction on the test portion and the control portion. However, the present disclosure is limited thereto.

For example, when the reaction membrane is a nitrocellulose membrane, the nitrocellulose membrane may be used as it is not treated. Alternatively, an entire nitrocellulose membrane may be wetted with a solution specially designed for nonspecific reaction or solution development and may be then dried.

In accordance with the present invention, the diagnosis unit is configured to diagnose a pregnancy related state of the subject, based on the ratio or the difference between the color intensity of the first test portion and the color intensity of the second test portion, wherein the color intensity of the first test portion is higher as an analyte concentration increases, and the color intensity of the second test portion is lower as the analyte concentration increases. However, the present disclosure is limited thereto.

In accordance with the present invention, the diagnosis unit is configured to diagnose a pregnancy related state of the subject, based on the ratio or the difference between the color intensity of the first test portion and the color intensity of the second test portion, wherein the color intensity of the first test portion occurs based on a sandwich immunoassay scheme, and the color intensity of the second test portion occurs based on a competitive immunoassay scheme. However, the present disclosure is limited thereto.

In one implementation embodiment of the present disclosure, each of the first test portion and/or the second test portion has an individual antigen or antibody coating. However, the present disclosure is limited thereto.

For example, the first test portion may have an antibody coating on the reaction membrane. The second test portion may have an antigen coating on the reaction membrane. However, the present disclosure is limited thereto.

In accordance with the present invention, the diagnosis unit is configured to: determine, for a first sample, a first ratio between a color intensity of the first test portion and a color intensity of the second test portion at a first time-point; determine, for a second sample, a second ratio between a color intensity of the first test portion and a color intensity of the second test portion at a second time-point; and diagnose the pregnancy related state of the subject based on the first ratio and the second ratio, wherein the second time-point is temporally subsequent to the first time-point. However, the present disclosure is limited thereto.

The ratio may be 'a color intensity of a second test line/a color intensity of a first test line'. However, the present disclosure is limited thereto.

In one implementation embodiment of the present disclosure, the diagnosis unit is configured to output normal information or abnormal information as information about the pregnancy related state, based on the first ratio and the second ratio. However, the present disclosure is limited thereto.

In one implementation embodiment of the present disclosure, the diagnosis unit is configured to: output the normal information when the first ratio is greater than the second ratio; or output the abnormal information when the first ratio is smaller than the second ratio. However, the present disclosure is limited thereto.

In one implementation embodiment of the present disclosure, the diagnosis unit is configured to: output the normal information when a value of the ratio gradually decreases from the first ratio to the second ratio as a time elapses from the first time-point to the second time-point; or output the abnormal information when a value of the ratio gradually increases from the first ratio to the second ratio as a time elapses from the first time-point to the second time-point. However, the present disclosure is limited thereto.

In one implementation embodiment of the present disclosure, the normal information indicates normal pregnancy or normal pregnancy maintenance, and the abnormal information indicates abnormal pregnancy or miscarriage. However, the present disclosure is limited thereto.

For example, when the color of the first test portion is rendered due to human chorionic gonadotropin (hCG) of the sample, and the color of the second test portion is rendered due to p3g (pregnanediol-3-glucuronide) as a type of progesterone and when the first ratio is greater than the second ratio, the normal information may be output.

Moreover, when the first test portion is colored due to human chorionic gonadotropin (hCG) of the sample, and the second test portion is colored due to p3g (pregnanediol-3-glucuronide) as a type of progesterone, and when the value of the ratio gradually decreases from the first ratio to the second ratio as a time elapses from the first time-point to the second time-point, the normal information related to a pregnancy may be output.

Conversely, when the color of the first test portion is rendered due to human chorionic gonadotropin (hCG) of the sample, and the color of the second test portion is rendered due to p3g (pregnanediol-3-glucuronide) as a type of progesterone and when the first ratio is smaller than the second ratio, the abnormal information may be output. Moreover, when the first test portion is colored due to human chorionic gonadotropin (hCG) of the sample, and the second test portion is colored due to p3g (pregnanediol-3-glucuronide) as a type of progesterone, and when the value of the ratio gradually increases from the first ratio to the second ratio as a time elapses from the first time-point to the second time-point, the abnormal information related to a pregnancy may be output.

The normal information as information about the state related to the pregnancy may include pregnancy, during pregnancy, maintenance of pregnancy, conception, fertilization, impregnation, or normal pregnancy. However, the present disclosure is limited thereto.

The abnormal information as information about the state related to the pregnancy may include abnormal pregnancy, abnormal ectopic pregnancy, requirement of visit to obstetrics and gynecology, risk of pregnancy, or miscarriage. However, the present disclosure is limited thereto.

Analyzing the color intensity of each of the first test portion and the second test portion may allow checking the presence of pregnancy, as well as the condition of ectopic pregnancy and miscarriage via hormone analysis, so that the subject may visit the hospital early to prevent an emergency situation.

In one implementation embodiment of the present disclosure, the diagnosis unit is configured to: output the normal information when the first ratio is smaller than the second ratio; or output the abnormal information when the first ratio is greater than the second ratio. However, the present disclosure is limited thereto.

In one implementation embodiment of the present disclosure, the diagnosis unit is configured to: output the normal information when a value of the ratio gradually increases from the first ratio to the second ratio as a time elapses from the first time-point to the second time-point; or output the abnormal information when a value of the ratio gradually decreases from the first ratio to the second ratio as a time elapses from the first time-point to the second time-point. However, the present disclosure is limited thereto.

In one implementation embodiment of the present disclosure, the normal information indicates that the subject is able to be pregnant, wherein the abnormal information indicates that the subject is not able to be pregnant or is in a menopause state. However, the present disclosure is limited thereto.

For example, when the color of the first test portion is rendered due to follicle stimulating hormone (FSH) of the sample, and the color of the second test portion is rendered due to by e3g (estrone-3-glucuronide) as a type of estrogen and when the first ratio is smaller than the second ratio, the normal information may be output.

Moreover, when the first test portion is colored due to follicle stimulating hormone (FSH) of the sample, and the second test portion is colored due to by e3g (estrone-3-glucuronide) as a type of estrogen, and when the value of the ratio gradually increases from the first ratio to the second ratio as a time elapses from the first time-point to the second time-point, the normal information related to a pregnancy may be output.

Conversely, when the color of the first test portion is rendered due to follicle stimulating hormone (FSH) of the sample, and the color of the second test portion is rendered due to by e3g (estrone-3-glucuronide) as a type of estrogen and when the first ratio is greater than the second ratio, the abnormal information may be output.

Moreover, when the first test portion is colored due to follicle stimulating hormone (FSH) of the sample, and the second test portion is colored due to by e3g (estrone-3-glucuronide) as a type of estrogen, and when the value of the ratio gradually decreases from the first ratio to the second ratio as a time elapses from the first time-point to the second time-point, the normal information related to a pregnancy may be output. The normal information is information indicating that the subject is able to be pregnant and may include non-menopause, pregnancy ability, being suspected to be amenorrhea due to other causes, or requirement of visit to obstetrics and gynecology. However, the present disclosure is limited thereto.

The abnormal information may include during menopause, menopause in progress, menopause, transition state to menopause, climacterium, amenorrhea, suspected of menopause in progress, or pregnancy inability. However, the present disclosure is limited thereto.

Analyzing the color intensity each of the first test portion and the second test portion may allow checking the situation of menopause, transition state to menopause, etc. due to hormonal changes. Thus, the subjects, in particular, women around the age of 50 may check the hormonal changes by themselves. When the menopause progresses, women may accept calmly this situation as a natural process of physical change. Thus, the subject may not be embarrassed with the physical and mental symptoms and may actively deal with the situation.

In one implementation embodiment of the present disclosure, the diagnosis unit is configured to: when the first ratio is a minimum value among a plurality of ratios including the first ratio and the second ratio, output time-point information corresponding to the first time-point as information about the pregnancy related state. However, the present disclosure is limited thereto.

In one implementation embodiment of the present disclosure, the time-point information is time-point information about ovulation. However, the present disclosure is limited thereto.

For example, when the first test portion is colored due to the luteinizing hormone (LH) of the sample and the second test portion is colored due to p3g (pregnanediol-3-glucuronide) as a type of progesterone, or e3g (estrone-3-glucuronide) as a type of estrogen, and when the first ratio is the minimum ratio among the plurality of ratios including the first ratio and the second ratio, ovulation state information corresponding to the first time-point may be displayed.

The ovulation state information may include ovulation, ovulation day, fertility period, ovulation period, or egg release. However, the present disclosure is limited thereto.

In one implementation embodiment of the present disclosure, the diagnosis unit is configured to: compare a plurality of ratios including the first ratio and the second ratio with each other; and when ratios below a threshold ratio last for a predetermined period, output the abnormal information. However, the present disclosure is limited thereto.

The threshold ratio may vary according to the color intensity of the diagnostic device.

The predetermined period may be a period for which the state of amenorrhea continuously progresses.

For example, when the first test portion is colored due to the luteinizing hormone (LH) of the sample, and the second test portion is colored by p3g (pregnanediol-3-glucuronide) as a type of progesterone, or e3g (estrone-3-glucuronide) as a type of estrogen, and when a state in which the ratio between the color intensity of the first test portion and the color intensity of the second test portion is 2.4 or smaller continues for 5 days or more, a polycystic ovary syndrome may be suspected and thus requirement of the visit to the gynecologist may be displayed. However, the present disclosure is limited thereto.

The abnormal information may include polycystic ovary syndrome, amenorrhea, rare menstruation, requirement of the visit to the gynecologist, ovulation disorder, rare ovulation, menstrual abnormality, or frequent menstruation. However, the present disclosure is limited thereto.

Prior art diagnostic methods may not read the polycystic ovary syndrome in which a state in which the concentration of the LH is above a certain concentration lasts. However, according to the present disclosure, analyzing the ratio of the color intensities according to concentrations of the LH and the estrogen or the progesterone may allow identifying the abnormal state of ovulation such as polycystic ovary syndrome to quickly recognize the need for hospital visit.

In particular, when only the concentration of the LH is analyzed using a conventional diagnostic method, the ovulation day of a person whose concentration of the LH is maintained at a low concentration cannot be read. However, according to the present disclosure, analyzing the ratio of color intensities according to the concentrations of the LH and the estrogen or the progesterone may allow determining the ovulation day based on the minimum value of the ratio value.

Moreover, according to the present disclosure, the device may analyze various hormone patterns by simultaneously analyzing a first substance and a second substance at a specific time-point and thus may predict and determine more accurately the state of the subject than the conventional diagnosis method based on the cut-off may.

In one implementation embodiment of the present disclosure, the state of the subject may be diagnosed using a method selected from the group consisting of immunochromatography, ELISA, radioimmunoassay, enzyme immunoassay, particle aggregation assay, chemiluminescence immunoassay, real-time polymerase reaction, flow cytometry, immunosensor, radial immunodiffusion, rocket immunoelectrophoresis, two-dimensional electrophoretic analysis, liquid chromatography-mass spectrometry (LC-MS), Western blot, and combinations thereof. However, the present disclosure is limited thereto.

In one implementation embodiment of the present disclosure, the diagnosis unit includes a light emitting unit and a light receiving unit. However, the present disclosure is limited thereto.

FIG. 3A and FIG. 3B are schematic diagrams of the diagnosis unit according to one implementation embodiment of the present disclosure.

Specifically, the first test portion 131 and the second test portion 132 may be disposed on the reaction unit 100. The diagnosis unit may include a light emitting unit 210 and a light receiving unit 221 and 222. However, the present disclosure is limited thereto.

In one implementation embodiment of the present disclosure, the light receiving unit measures the color intensity based on an amount of light reflected from the reaction unit when the diagnosis unit irradiates light on the reaction unit. However, the present disclosure is limited thereto.

The diagnostic device may operate at low power. The term 'low power' refers to a voltage as realized using a battery. For example, the voltage may be 9 V or less, 8 V or less, 7 V or less, 6 V or less, preferably 5 V or less, and more preferably 3 V or less.

The diagnostic device may be of a miniature size. The term 'miniature' means a size and a weight at which the device may be held lightly with one hand. Examples of the diagnostic device of the miniature size may include a blood glucose meter and a digital pregnancy test. The diagnostic device may be of a very small size and weight commonly used in the art.

The diagnostic device may have a short side length of 10 cm or less, 9 cm or less, and 8 cm or less. For example, the short side length may be 1 cm to 10 cm, 1 cm to 9 cm, 1 cm to 8 cm, 2 cm to 10 cm, 2 cm to 9 cm, 2 cm to 8 cm. However, the present disclosure is limited thereto.

Moreover, the diagnostic device may have a long side length of 15 cm or less, 14 cm or less, 13 cm or less, 12 cm or less, 11 cm or less, 10 cm or less. For example, the long side length may be 1 cm to 15 cm, 1 cm to 14 cm, 1 cm to 13 cm, 1 cm to 12 cm, 1 cm to 11 cm, 1 cm to 10 cm. However, the present disclosure is limited thereto.

Moreover, the diagnostic device may have a height of 4.0 cm or less, 3.5 cm or less, 3.0 cm or less, 2.5 cm or less. For example, the height may be 1.0 cm to 4.0 cm, 1.0 cm to 3.5 cm, 1.0 cm to 3.0 cm, 1.0 cm to 2.5 cm. However, the present disclosure is limited thereto.

The weight of the diagnostic device may be 400 g or less, 350 g or less, 300 g or less, 250 g or less, 200 g or less, for example, 100 g to 400 g, 100 g to 350 g, 100 g to 300 g, 100 g to 250 g, 100 g to 200 g. However, the present disclosure is limited thereto.

The light emitting unit may include a light source that generates light. However, the present disclosure is limited thereto.

The light source may be various light sources known in the art. For example, the light source may include a light source selected from the group consisting of LED (light emitting diode), laser, tungsten lamp and combinations thereof. However, the present disclosure is limited thereto. Preferably, the light source may be LED.

The light source may emit monochromatic light. For example, the light may be red-based or green-based monochromatic light. When the light source emits monochromatic light, the light has candela sufficient for analysis, and power consumption is not large even after repeated measurements.

The red-based monochromatic light may have a wavelength of 600 nm to 670 nm, 600 nm to 665 nm, 600 nm to 660 nm, 600 nm to 655 nm, 600 nm to 650 nm, 605 nm to 670 nm, 605 nm to 665 nm, 605 nm to 660 nm, 605 nm to 655 nm, 605 nm to 650 nm, 610 nm to 670 nm, 610 nm to 665 nm, 610 nm to 660 nm, 610 nm to 655 nm, 610 nm to 650 nm, 615 nm to 670 nm, 615 nm to 665 nm, 615 nm to 660 nm, 615 nm to 655 nm, 615 nm to 650 nm, 620 nm to 670 nm, 620 nm to 665 nm, 620 nm to 660 nm, 620 nm to 655 nm, 620 nm to 650 nm, preferably, 620 nm to 650 nm.

The green-based monochromatic light may have a wavelength of 500 nm to 580 nm, 500 nm to 570 nm, 500 nm to 560 nm, 500 nm to 550 nm, 510 nm to 580 nm, 510 nm to 570 nm, 510 nm to 560 nm, 510 nm to 550 nm, 520 nm to 580 nm, 520 nm to 570 nm, 520 nm to 560 nm, 520 nm to 550 nm, preferably, 520 nm to 550 nm.

At least one light emitting unit included in the light emitting units irradiates light to the reaction unit included in the diagnostic device. At least one further light emitting unit may irradiate light to identification portions (the test portion and the control portion) included in the diagnostic device.

The diagnostic device may include a first light emitting unit that irradiates light to the reaction unit and a second light emitting unit that irradiates light to the identification portion, wherein the first and second light emitting units are different from each other.

Light beams irradiated to one or more zones selected from the group consisting of a test portion, a control portion and a background zone included in the reaction unit may come from the same or different light emitting units. However, the present disclosure is limited thereto.

The light emitting unit that irradiates light to the identification portion of the diagnostic device may include at least one or more light emitting units. For example, the number of at least one or more light emitting units may correspond to the number of identification regions included in the identification portion. However, the present disclosure is limited thereto.

For example, the same first light emitting unit may irradiates light to the test portion and the background zone. The same second light emitting unit may irradiate light to the control portion and the background zone. The first light emitting unit and the second light emitting unit may be different from each other. However, the present disclosure is limited thereto.

Moreover, the light emitting units to irradiate light beams on the test portion, the background zone and the control portion, respectively, may be a first light emitting unit, a second light emitting unit, and a third light emitting unit, respectively, and may be different from each other. However, the present disclosure is limited thereto.

The light emitting unit may additionally include a light distributer. However, the present disclosure is limited thereto. When using the light distributer, one light emitting unit may irradiate light to the test portion, the control portion, and the background zone under an equal condition.

The diagnosis unit is equipped with at least two light receiving units. At least one light receiving unit may receive light reflected from the reaction unit. At least one further light receiving unit may receive light reflected from the identification portion. However, the present disclosure is limited thereto.

The light receiving unit for receiving light reflected from the reaction unit and the light receiving unit for receiving light reflected from the identification portion of the diagnosis unit may be different from each other. However, the present disclosure is limited thereto.

The light receiving unit that receives light reflected from the identification portion of the diagnosis unit may include at least one or more light receiving units. For example, the number thereof may correspond to the number of identification regions included in the identification portion.

For example, the same first light receiving unit may receive light reflected from the test portion and the background zone. The same second light receiving unit may receive light reflected from the control portion and the background zone. The first light receiving unit and the second light receiving unit may be different from each other. However, the present disclosure is limited thereto.

Moreover, the light receiving units that receive light beams reflected from the test portion, the background zone and the control portion, respectively may be a first light receiving unit, a second light receiving unit, and a third light receiving unit, respectively, and may be different from each other. However, the present disclosure is limited thereto.

The light receiving units for receiving light beams reflected from one or more zones selected from the group consisting of the test portion, the control portion, and the background zone included in the reaction unit may be the same or different light receiving units.

The light receiving unit may include one selected from the group consisting of photodiode, phototransistor, photoresistor, and combinations thereof. However, the present disclosure is limited thereto.

In order to measure the color intensity of the reaction unit, the light emitting unit of the diagnosis unit may include a first light emitting unit and a second light emitting unit, and the light receiving unit may include a first light receiving unit, a second light receiving unit and a third light receiving unit. However, the present disclosure is limited thereto.

Moreover, light from the first light source may be irradiated to the test portion and the background zone. Light from the second light emitting unit may be irradiated to the background zone and the control portion. The light beams reflected from the test portion, the background zone and the control portion may be detected by the first light receiving unit, the second light receiving unit and the third light receiving unit, respectively. However, the present disclosure is limited thereto.

In addition to the reaction unit, the diagnosis unit, the light emitting unit, and the light receiving unit as described above, the diagnostic device may further include a microcontroller such as a CPU (central processing unit), flash memory, ADC (analog-to-digital converter) and a comparator, a voltage regulator, and/or the display, such as an LCD, for measurement control and calculation and output. However, the present disclosure is limited thereto.

Moreover, the diagnostic device may additionally include a detector switch, a battery and/or a serial programming connector. However, the present disclosure is limited thereto.

The diagnostic device may diagnose various diseases and health state by changing and measuring the reaction unit according to the purpose to be diagnosed.

According to one implementation embodiment of the present disclosure, the device may further include a wireless communication module. However, the present disclosure is limited thereto.

The wireless communication module may wirelessly transmit information obtained by analyzing the color intensity of the reaction unit to the electronic device.

The electronic device may include a smartphone, tablet, laptop, computer, etc. For example, as long as the electronic device includes wireless communication protocols such as Bluetooth, Wi-Fi, NFC (Near Field Communication), the electronic device may not be limited particularly.

For example, the user may constantly check the hormonal changes over time with the smartphone and may know related information additionally. This may improve the quality of life of women by the women identifying physical and mental changes by themselves and preventing or coping with them in advance.

The second aspect of the present disclosure relates to a diagnostic device set, including the diagnostic device and an analysis reader into which the diagnostic device is inserted.

The diagnostic device set may be another expression of the diagnostic device as described above in the first aspect of the present disclosure. That is, the diagnostic device set may be the same as the diagnostic device.

The analysis reader may include a light emitting unit and a light receiving unit. However, the present disclosure is limited thereto.

The analysis reader may equally act as the diagnosis unit as described above.

FIG. 4A and FIG. 4B are schematic diagrams of a diagnostic device set according to one implementation embodiment of the present disclosure.

Specifically, the diagnostic device set may include a diagnosis unit 200 or an analysis reader capable of measuring the color intensity rendered in the reaction unit 100, separately. According to this embodiment, the reaction unit 100 may be inserted into a separate diagnosis unit 200 or analysis reader that is spaced apart therefrom, while the subject sample is received in the reaction unit 100. In connection with this embodiment, the descriptions omitted in connection with the reaction unit 100, the diagnosis unit 200, and the analysis reader may refer to those described with reference to FIG. 1 to FIG. 3 above.

We illustrate the present disclosure in more detail based on following embodiment. However, the following embodiments are for illustrative purposes only and are not intended to limit the scope of the present disclosure.

### [Embodiment 1]

The reaction unit 100 was formed such that the first test portion exhibited the color intensity due to hCG (human chorionic gonadotropin) and the second test portion exhibited the color intensity due to the progesterone metabolite P3G.

### [Embodiment 2]

The reaction unit 100 was formed so that the first test portion exhibited color intensity due to follicle stimulating hormone (FSH) and the second test portion exhibited color intensity due to the estrogen metabolite E3G.

### [Embodiment 3]

The reaction unit 100 was formed such that the first test portion exhibited color intensity due to luteinizing hormone (LH), and the second test portion exhibited color intensity due to estrogen metabolite E3G, and the third test portion (control portion) exhibited color intensity due to progesterone metabolite P3G.

### [Embodiment 4]

The reaction unit 100 was formed such that the first test portion exhibited color intensity due to luteinizing hormone (LH), the second test portion exhibited color intensity due to progesterone metabolite P3G, and the third test portion (control portion) exhibited color intensity due to estrogen metabolite E3G.

Moreover, in Embodiment 1 to Embodiment 4, the diagnosis unit 200 or analysis reader as described above detects the color intensity rendered in the reaction unit 100, and may diagnose the subject states according to the detection result.

### [Experimental Embodiment]

The characteristics appearing in the reaction unit 100 prepared in the Embodiment 1 were identified. The results are shown as FIG. 5, FIG. 6A and FIG. 6B.

FIG. 5 is a graph showing change in a color intensity according to a hormone concentration of the reaction unit 100 according to one embodiment of the present disclosure.

Referring to FIG. 5, in the second test portion T2, due to the competitive immunoassay scheme, and the color intensity decreased as the concentration of the analyte increased. In the first test portion T1, due to the sandwich immunoassay scheme, the color intensity increased as the concentration of the analyte increased.

FIG. 6A is a real color picture according to a concentration of the progesterone metabolite of the reaction unit 100 according to one embodiment of the present disclosure. FIG. 6B is a real color picture according to a concentration of human chorionic gonadotropin of the reaction unit 100 according to one embodiment of the present disclosure.

Specifically, FIG. 6A shows a color picture when the hCG is maintained at 0 IU/ml, and when the P3G is in the concentration range of 0 µg/ml to 500 µg/ml. FIG. 6B shows a color picture when the P3G is maintained at 0 µg/ml, and when the hCG is in a concentration range of 0 mIU/ml to 200 mIU/ml.

The reaction unit 100 prepared in Embodiment 1 was used to identify absence or presence of pregnancy. The results are shown as FIG. 7 to FIG. 10.

FIG. 7A is a graph showing a color intensity due to hormone over time after menstruation of the reaction unit 100 according to one embodiment of the present disclosure. FIG. 7B is a graph showing a ratio of hormone in FIG. 7A. FIG. 7C shows a real color picture and results according to a concentration of hormone in the reaction unit 100 according to one embodiment of the present disclosure.

FIG. 7A to FIG. 7C are the results of a 29-year-old woman trying to become pregnant.

According to the results shown in FIG. 7A to FIG. 7C, the reaction unit 100 exhibited the color intensity of the first test portion T1 and the color intensity of the second test portion T2 over time. In this connection, the color intensity of the first test portion T1 may be determined based on the concentration of analyte hCG. The color intensity of the second test portion T2 may be determined based on the concentration of analyte P3G. The diagnosis unit may detect that the ratio of the color intensity of the second test portion T2 to the color intensity of the first test portion T1 is gradually decreasing. Thus, the diagnosis unit may display the normal information related to the state of pregnancy. In this connection, the normal information may mean normal pregnancy or maintenance of pregnancy.

That is, the reaction unit 100 may diagnose the state of the subject by detecting and analyzing the result values shown in FIG. 7A to FIG. 7C, and may analyze the results shown in FIG. 7A to FIG. 7C and display the pregnancy state based on the analysis result.

FIG. 8A is a graph showing a color intensity due to hormone over time after menstruation of the reaction unit 100 according to one embodiment of the present disclosure. FIG. 8B is a graph showing a ratio of hormone in FIG. 8A. FIG. 8C shows a real color picture and results according to a concentration of hormone of the reaction unit 100 according to one embodiment of the present disclosure.

FIG. 8A to FIG. 8C are the results of a 30-year-old woman in the middle of the pregnancy.

According to the results shown in FIG. 8A to FIG. 8C, the reaction unit 100 exhibits the color intensity of the first test portion T1 and the color intensity of the second test portion T2 over time. In this connection, the color intensity of the first test portion T1 may be determined based on the concentration of analyte hCG. The color intensity of the second test portion T2 may be determined based on the concentration of analyte P3G. The diagnosis unit may detect that the ratio of the color intensity of the second test portion T2 to the color intensity of the first test portion T1 is gradually decreasing. Thus, the diagnosis unit may display normal information related to the state of pregnancy. In this connection, the normal information may mean normal pregnancy or maintenance of pregnancy.

The diagnosis unit 200 or analysis reader may diagnose the state of the subject by detecting and analyzing the result values shown in FIG. 8A to FIG. 8C via the reaction unit 100 according to Embodiment 1, and may analyze the results shown in FIG. 8A to FIG. 8C, and may display the normal pregnancy state.

FIG. 9A is a graph showing a color intensity due to hormone over time after menstruation of the pregnancy check diagnostic device according to one embodiment of the present disclosure. FIG. 9B is a graph showing a ratio of hormone in FIG. 9A. FIG. 9C shows a real color picture and results according to a concentration of hormones in the pregnancy check diagnostic device according to one embodiment of the present disclosure.

FIG. 9A to FIG. 9C are the results of a woman who was 38 years old and was identified to be pregnant.

According to the results shown in FIG. 9A to FIG. 9C, the reaction unit 100 exhibits the color intensity of the first test portion T1 and the color intensity of the second test portion T2 over time. In this connection, the color intensity of the first test portion T1 may be determined based on the concentration of analyte hCG. The color intensity of the second test portion T2 may be determined based on the concentration of analyte P3G. The diagnosis unit may detect that the ratio of the color intensity of the second test portion T2 to the color intensity of the first test portion T1 is gradually increasing. Thus, the diagnosis unit may display abnormal information related to the state of pregnancy. In this connection, the abnormal information may mean abnormal pregnancy, miscarriage, or ectopic pregnancy. Moreover, it may be identified that with considering the number of weeks of pregnancy, an ectopic pregnancy is suspected and this requires visit to obstetrics and gynecology.

That is, the pregnancy check diagnostic device may diagnose the state of the subject by detecting and analyzing the result values shown in FIG. 9A to FIG. 9C, and may analyze the results shown in FIG. 9A to FIG. 9C, and may display an ectopic pregnancy or abnormal pregnancy state.

FIG. 10A is a graph showing a color intensity due to hormone over time after menstruation of a pregnancy check diagnostic device according to one embodiment of the present disclosure. FIG. 10B is a graph showing a ratio of hormone in FIG. 10A. FIG. 10C shows a real color picture and results according to a concentration of hormones in the pregnancy check diagnostic device according to one embodiment of the present disclosure.

FIG. 10A to FIG. 10C are the results of a woman who was identified to be pregnant and 32 years old.

According to the results shown in FIG. 10A to FIG. 10C, the reaction unit 100 exhibits the color intensity of the first test portion T1 and the color intensity of the second test portion T2 over time. In this connection, the color intensity of the first test portion T1 may be determined based on the concentration of analyte hCG. The color intensity of the second test portion T2 may be determined based on the concentration of analyte P3G. The diagnosis unit may detect that the ratio of the color intensity of the second test portion T2 to the color intensity of the first test portion T1 is gradually increasing. Thus, the diagnosis unit may display abnormal information related to the state of pregnancy. In this connection, the abnormal information may mean abnormal pregnancy, miscarriage or pregnancy risk.

That is, the pregnancy check diagnostic device may diagnose the state of the subject by detecting and analyzing the result values shown in FIG. 10A to FIG. 10C, and may analyze the results presented in the FIG. 10A to FIG. 10C and may display the risk state of pregnancy.

The characteristics of the diagnostic device for the menopause check prepared in the Embodiment were identified. The results are shown in FIG. 11, FIG. 12A and FIG. 12B.

FIG. 11 is a graph showing change in a color intensity according to a hormone concentration in the diagnostic device for menopause check according to one embodiment of the present disclosure.

Referring to FIG. 11, in the second test portion T2, due to the competitive immunoassay scheme, the color intensity decreased as the concentration of the analyte increased. In the first test portion T1, due to the sandwich immunoassay scheme, the color intensity increased as the concentration of the analyte increased.

FIG. 12A is a real color picture according to a concentration of estrogen metabolites in the diagnostic device for menopause check according to one embodiment of the present disclosure. FIG. 12B is a real color picture according to a concentration of follicle stimulating hormone in the diagnostic device for menopause check according to one embodiment of the present disclosure.

Specifically, FIG. 12A shows a color picture when the FSH is maintained at 0 IU/ml, and when the E3G is in the concentration range of 0 ng/ml to 500 ng/ml. FIG. 12B shows a color picture when the E3G is maintained at 0 ng/ml, and when the FSH concentration ranges from 0 mIU/ml to 100 mIU/ml.

The diagnostic device for menopause check prepared in Embodiment 2 was used to check whether menopause progressed. The results are shown as FIG. 13A to FIG. 13C and FIG. 14A to FIG. 14C.

FIG. 13A is a graph showing a color intensity due to hormone over time in the diagnostic device for menopause check according to one embodiment of the present disclosure. FIG. 13B is a graph showing a ratio of hormone in FIG. 13A. FIG. 13C shows a real color picture and results according to a concentration of hormones in the diagnostic device for menopause check according to one embodiment of the present disclosure.

FIG. 13A to FIG. 13C are the results of a 54-year-old woman whose amenorrhea state lasted for 2 months.

According to the results shown in FIG. 13A to FIG. 13C, the reaction unit 100 exhibits the color intensity of the first test portion T1 and the color intensity of the second test portion T2 over time. In this connection, the color intensity of the first test portion T1 may be determined based on the concentration of analyte FSH. The color intensity of the second test portion T2 may be determined based on the concentration of analyte E3G. The diagnosis unit may detect that the ratio of the color intensity of the second test portion T2 to the color intensity of the first test portion T1 is gradually decreasing. Thus, the diagnosis unit may display abnormal information related to the state of pregnancy. In this connection, the abnormal information may mean pregnancy inability or menopause.

That is, the diagnostic device for menopause check may diagnose the state of the subject by detecting and analyzing the results shown in FIG. 13A to FIG. 13C and may analyze the results shown in the FIG. 13A to FIG. 13C and may display the state which menopause progressed.

FIG. 14A is a graph showing a color intensity due to hormone over time in the diagnostic device for menopause check according to one embodiment of the present disclosure. FIG. 14B is a graph showing a ratio of hormone in FIG. 14A. FIG. 14C shows a real color picture and results according to a hormone concentration in the diagnostic device for menopause check according to one embodiment of the present disclosure.

FIG. 14A to FIG. 14C are the results of a 48-year-old woman who experienced amenorrhea last month.

According to the results shown in FIG. 14A to FIG. 14C, the reaction unit 100 exhibits the color intensity of the first test portion T1 and the color intensity of the second test portion T2 over time. In this connection, the color intensity of the first test portion T1 may be determined based on the concentration of analyte FSH. The color intensity of the second test portion T2 may be determined based on the concentration of analyte E3G. The diagnosis unit may detect that the ratio of the color intensity of the second test portion T2 to the color intensity of the first test portion T1 is gradually increasing. Thus, the diagnosis unit may display normal information related to the state of pregnancy. In this connection, the normal information may mean that the subject is able to be pregnant or is not in the menopause state. Therefore, it may be determined that amenorrhea is due to other causes. She identified that a visit to obstetrics and gynecology was necessary.

That is, the diagnostic device for menopause check may diagnose the state of the subject by detecting and analyzing the results shown in FIG. 14A to FIG. 14C, and may analyze the results shown in the FIG. 14A to FIG. 14C, and may display that the subject is not in the menopause state or may indicate that the visit to obstetrics and gynecology is necessary when amenorrhea persists.

The characteristics of the diagnostic device for ovulation check prepared in Embodiment 3 were identified. The results are shown as FIG. 15, FIG. 16A and FIG. 16B.

FIG. 15 is a graph showing change in a color intensity according to a hormone concentration in the diagnostic device for ovulation check according to one embodiment of the present disclosure.

Referring to FIG. 15, in the second test portion T2, due to the competitive immunoassay scheme, the color intensity decreased as the concentration increased. In the first test portion T1, due to the sandwich immunoassay scheme, the color intensity increased as the concentration increased.

FIG. 16A is a real color picture according to a concentration of luteinizing hormone in the diagnostic device for ovulation check according to one embodiment of the present disclosure. FIG. 16B is a real color picture according to a concentration of estrogen metabolites in the diagnostic device for ovulation check according to one embodiment of the present disclosure.

Specifically, FIG. 16A shows a color picture when the P3G and the E3G are maintained at 0 ng/ml, and when the LH is in the concentration range of 0 mIU/ml to 500 mIU/ml. FIG. 6B shows a color picture when the P3G is maintained at 0 µg/ml and the LH at 0 mIU/ml, and when the E3G has a concentration range of 0 ng/ml to 500 ng/ml.

The diagnostic device for ovulation check as prepared in Embodiment 3 was used to identify the ovulation day. The results are shown in FIG. 17 to FIG. 19.

FIG. 17A is a graph showing a color intensity due to hormone over time after menstruation in a diagnostic device for ovulation check according to one embodiment of the present disclosure. FIG. 17B is a graph showing a ratio of hormone in FIG. 17A. FIG. 17C shows a real color picture and results according to a concentration of hormones in the diagnostic device for ovulation check according to one embodiment of the present disclosure.

FIG. 17A to FIG. 17C are the results of a 34-year-old woman trying to become pregnant.

According to the results shown in FIG. 17A to FIG. 17C, the reaction unit 100 exhibits the color intensity of the first test portion T1 and the color intensity of the second test portion T2 over time. In this connection, the color intensity of the first test portion T1 may be determined based on the concentration of analyte LH. The color intensity of the second test portion T2 may be determined based on the concentration of analyte E3G. The diagnosis unit may detect the minimum value of the ratio of the color intensity of the second test portion T2 to the color intensity of the first test portion T1. Thus, the diagnosis unit may display state information related to the state of pregnancy. In this connection, the state information may mean ovulation. Accordingly, the 13-th day after menstruation when the ratio of the color intensity of the second test portion T2 to the color intensity of the first test portion T1 is minimum value may be displayed as the ovulation day. When the subject checks the ovulation day, she may check the fertility period and increase the probability of success of the pregnancy.

That is, the diagnostic device for ovulation check may detect and analyze the result values shown in FIG. 17A to FIG. 17C to diagnose the state of the subject and may analyze the results shown in the FIG. 17A to FIG. 17C and may display the ovulation state or fertility period.

FIG. 18A is a graph showing a color intensity of hormones over time after menstruation in a diagnostic device for ovulation check according to one embodiment of the present disclosure. FIG. 18B is a graph showing a ratio of hormone in FIG. 18A. FIG. 18C shows a real color picture and results according to a concentration of hormone in the diagnostic device for ovulation check according to one embodiment of the present disclosure.

FIG. 18A to FIG. 18C are the results of a 36-year-old woman trying to become pregnant.

According to the results shown in FIG. 18A to FIG. 18C, the reaction unit 100 exhibits the color intensity of the first test portion T1 and the color intensity of the second test portion T2 over time. In this connection, the color intensity of the first test portion T1 may be determined based on the concentration of analyte LH. The color intensity of the second test portion T2 may be determined based on the concentration of analyte E3G. The diagnosis unit may detect the minimum value of the ratio of the color intensity of the second test portion T2 to the color intensity of the first test portion T1. Thus, the diagnosis unit may display state information related to the state of pregnancy. In this connection, the state information may mean ovulation. Therefore, the 13-th day after menstruation when the ratio of the color intensity of the second test portion T2 to the color intensity of the first test portion T1 is minimum value may be displayed as an ovulation day. When the subject checks the ovulation day, she may check the fertility period and increase the probability of success of the pregnancy. That is, the diagnostic device for ovulation check may detect and analyze the result values shown in FIG. 18A to FIG. 18C to diagnose the state of the subject, may analyze the results shown in the FIG. 18A to FIG. 18C and may display the ovulation state or fertility period.

FIG. 19A is a graph showing a color intensity of hormones over time after menstruation in a diagnostic device for ovulation check according to one embodiment of the present disclosure. FIG. 19B is a graph showing a ratio of hormone in FIG. 19A. FIG. 19C shows a real color picture and results according to a concentration of hormones in the diagnostic device for ovulation check according to one embodiment of the present disclosure.

FIG. 19A to FIG. 19C are the results of a 27-year-old woman.

According to the results shown in FIG. 19A to FIG. 19C, the reaction unit 100 exhibits the color intensity of the first test portion T1 and the color intensity of the second test portion T2 over time. In this connection, the color intensity of the first test portion T1 may be determined based on the concentration of analyte LH. The color intensity of the second test portion T2 may be determined based on the concentration of analyte E3G. The diagnosis unit may compare the ratio of the color intensity of the first test portion T1 to the color intensity of the second test portion T2 and may detect a case where ratios below the threshold ratio last for a predetermined period. The threshold ratio value of the reaction unit 100 prepared in this embodiment may be 2.4, and the predetermined period may be 5 days. Thus, the diagnosis unit may display state information related to the state of pregnancy. In this connection, the state information may mean abnormal information. According to the results shown in this embodiment, it was found that the state in which the ratio of the color intensity of the second test portion T2 to the color intensity of the first test portion T1 was 2.4 or lower continued for 5 days. If the amenorrhea state persists for several months based on the results, the subject may be suspected of polycystic ovary syndrome. Therefore, it may be identified that the subject should visit an obstetrics and gynecology.

That is, the diagnostic device for ovulation check may diagnose the state of the subject by detecting and analyzing the result values shown in FIG. 19A to FIG. 19C. The diagnostic device for ovulation check may analyze the results shown in the FIG. 19A to FIG. 19C and may display the suspected state of polycystic ovary syndrome.

In particular, when only the concentration of the LH is analyzed using a conventional diagnostic method, the ovulation day of a person whose concentration of the LH is maintained at a low concentration cannot be read. However, according to the present disclosure, analyzing the ratio of color intensities according to the concentrations of the LH and the estrogen or the progesterone may allow determining the ovulation day based on the minimum value of the ratio value.

The characteristics of the diagnostic device for ovulation check as prepared in Embodiment 4 were identified. The results are shown as FIG. 20, FIG. 21A and FIG. 21B.

FIG. 20 is a graph showing the change in a color intensity according to a hormone concentration in the diagnostic device for ovulation check according to one embodiment of the present disclosure.

Referring to FIG. 20, in the second test portion P3G T2, due to the competitive immunoassay scheme, the color intensity decreased as the concentration increased. In the first test portion LH T1, due to the sandwich immunoassay scheme, the color intensity increased based on the increasing concentration.

FIG. 21A is a real color picture according to a concentration of luteinizing hormone in the diagnostic device for ovulation check according to one embodiment of the present disclosure. FIG. 21B is a real color picture according to a concentration of progesterone metabolites in the diagnostic device for ovulation check according to one embodiment of the present disclosure.

Specifically, FIG. 21A shows a color picture when the P3G and the E3G are maintained at 0 ng/ml, and when the LH is in the concentration range of 0 mIU/ml to 500 mIU/ml. FIG. 21B shows a color picture when the E3G is maintained at 0 ng/ml and the LH maintained at 0 IU/ml, and when the P3G is in a concentration range of 0 µg/ml to 50 µg/ml.

The diagnostic device for ovulation check as prepared in Embodiment 4 was used to identify the ovulation day. The results are shown as FIG. 22 to FIG. 23.

FIG. 22A is a graph showing a color intensity due to hormone over time after menstruation in a diagnostic device for ovulation check according to one embodiment of the present disclosure. FIG. 22B is a graph showing a ratio of hormone in FIG. 22A. FIG. 22C shows a real color picture and results according to a concentration of hormone in the diagnostic device for ovulation check according to one embodiment of the present disclosure.

FIG. 22A to FIG. 22C are the results of a 34-year-old woman trying to become pregnant.

According to the results shown in FIG. 22A to FIG. 22C, the reaction unit 100 exhibits the color intensity of the first test portion T1 and the color intensity of the second test portion T2 over time. In this connection, the color intensity of the first test portion T1 may be determined based on the concentration of analyte LH. The color intensity of the second test portion T2 may be determined based on the concentration of analyte P3G. The diagnosis unit may detect the minimum value of the ratio of the color intensity of the second test portion T2 to the color intensity of the first test portion T1. Thus, the diagnosis unit may display state information related to the state of pregnancy. In this connection, the state information may mean ovulation. Therefore, the 13-th day after menstruation when the ratio of the color intensity of the second test portion T2 to the color intensity of the first test portion T1 has the minimum value may be displayed as an ovulation day. When the subject checks the ovulation day, she may check the fertility period and increase the probability of success of the pregnancy.

That is, the diagnostic device for ovulation check may diagnose the state of the subject by detecting and analyzing the result values shown in FIG. 22A to FIG. 22C, and may analyze the results shown in FIG. 22A to FIG. 22C and may display ovulation state or fertility period.

FIG. 23A is a graph showing a color intensity due to hormone over time after menstruation in a diagnostic device for ovulation check according to one embodiment of the present disclosure. FIG. 23B is a graph showing a ratio of hormone in FIG. 23A. FIG. 23C shows a real color picture and results according to a concentration of hormone in the diagnostic device for ovulation check according to one embodiment of the present disclosure.

FIG. 23A to FIG. 23C are the results of a 36-year-old woman trying to become pregnant.

According to the results shown in FIG. 23A to FIG. 23C, the reaction unit 100 exhibits the color intensity of the first test portion T1 and the color intensity of the second test portion T2 over time. In this connection, the color intensity of the first test portion T1 may be determined based on the concentration of analyte LH. The color intensity of the second test portion T2 may be determined based on the concentration of analyte P3G. The diagnosis unit may detect the minimum value of the ratio of the color intensity of the second test portion T2 to the color intensity of the first test portion T1. Thus, the diagnosis unit may display state information related to the state of pregnancy. In this connection, the state information may mean ovulation. Therefore, the 13-th day after menstruation when the ratio of the color intensity of the second test portion T2 to the color intensity of the first test portion T1 has the minimum value may be displayed as an ovulation day. When the subject checks the ovulation day, she may check the fertility period and increase the probability of success of the pregnancy.

That is, the diagnostic device for ovulation check may diagnose the state of the subject by detecting and analyzing the result values shown in FIG. 23A to FIG. 23C and may analyze the results shown in the FIG. 23A to FIG. 23C and may display the ovulation state or fertility period.

In the prior art, when the LH was maintained at a concentration lower than the cut-off, this may not be detected. Thus, the ovulation day could not be identified. However, based on the results shown in FIG. 23A to FIG. 23C, the reaction unit may detect that the LH is maintained at a concentration lower than the normal cut-off. That is, the reaction unit 100 may detect the ovulation day based on the minimum value of the ratio as identified by analyzing the ratio of the color intensity of the second test line to the color intensity of the first test line.

A third aspect of the present disclosure provides a diagnostic method comprising: receiving a sample by a reaction unit; detecting, by a diagnosis unit, a color intensity of each of at least two test portions included in the reaction unit; and diagnosing, by the diagnosis unit, a subject state, based on a ratio or a difference between a color intensity of a first test portion and a color intensity of the second test portion.

FIG. 24 is a flowchart of a part of the diagnostic method according to one implementation embodiment of the present disclosure. That is, the flow chart shown through FIG. 24 is a flow chart of a part of the diagnostic method according to the third aspect of the present invention as defined with appended independent claim 23. The diagnostic method as described via FIG. 24 may be performed by the diagnostic device 1 or the diagnostic device set 2 as described above. Therefore, the descriptions of the diagnostic device 1 or the diagnostic device set 2 as made via FIG. 1 to FIG. 24 may also be applied to FIG. 24.

First, a sample is received in the reaction unit S 100.

The reaction unit may include the sample pad receiving the sample, the conjugate pad, the reaction membrane having at least two or more test portions, and the wicking pad. However, the present disclosure is limited thereto.

The sample pad may refer to a pad in which a sample to be tested or a solution to be analyzed is loaded and absorbed.

The conjugate pad may have a colored particle that reacts with the sample absorbed by the sample pad.

The colored particles of the conjugate pad move to the reaction membrane where the test portion and the control portion exhibit a result of detection based on the color.

The wicking pad may perform final absorption of the sample passing through the reaction membrane.

Subsequently, the diagnosis unit may detect color intensities of at least two or more test portions as appearing in the reaction unit S200.

Details about the two or more test portions disposed on the reaction membrane may be consistent with the contents of the test portion of the reaction unit as disposed in the diagnostic device as described above with reference to the first aspect of the present disclosure.

Subsequently, the diagnosis unit may diagnose the subject state based on a ratio or a difference between the color intensity of the first test portion and the color intensity of the second test portion S300. The diagnostic method using the diagnostic device 1 or the diagnostic device set 2 may be used in various fields such as diagnosis and prediction of disease, health care, paternity test, constitution analysis, fermentation engineering, biotechnology, food safety test, environmental analysis, cosmetic analysis and compound analysis.

The diagnostic method according to one implementation embodiment of the present disclosure is implemented in the form of program instructions that may be executed through various computer means and may be recorded in a computer-readable medium. The computer-readable medium may include program instructions, data files, data structures, or the like alone or in combination with each other. The program instructions recorded in the medium may be specially designed and configured for the present disclosure, or may be known by those skilled in computer software. Examples of computer-readable recording media include magnetic media such as hard disks, floppy disks and magnetic tapes, optical recording media such as CD-ROMs, DVDs, magnetic-optical media such as floptical disks, and a hardware apparatus specifically configured to store and execute program instructions such as ROM, RAM, and flash memory. Examples of program instructions include not only machine language codes created by a compiler, but also high-level language codes that may be executed by a computer using an interpreter. The hardware apparatus as described above may be configured to operate as one or more software modules to perform the operations of the present disclosure, or vice versa.

The above description of the present disclosure is for illustration only. Those of ordinary skill in the technical field to which the present disclosure belongs may understand that the description may be easily transformed into other specific forms without changing the technical idea or essential characteristics of the present disclosure. Therefore, the embodiments as described above are illustrative in all respects and should be understood as non-limiting. For example, each component described as a single type may be implemented in a distributed manner. Similarly, components described as a distributed type may be implemented in a combined form.

The scope of the present invention is limited only by the claims below, rather than the detailed description.

## Claims

1. A diagnostic device (1) for diagnosing a state of a subject based on a ratio or a difference between a color intensity of a first test portion (131) and a color intensity of a second test portion (132) in a reaction unit (100) receiving a sample therein,
wherein the device includes:
the reaction unit (100) receiving the sample therein and having two or more test portions; and
a diagnosis unit (200) for diagnosing the state of the subject based on the ratio or the difference between the color intensity of the first test portion (131) and the color intensity of the second test portion (132);
wherein the diagnosis unit (200) is configured to diagnose a pregnancy related state of the subject, based on the ratio or the difference between the color intensity of the first test portion (131) and the color intensity of the second test portion (132), wherein the color intensity of the first test portion (131) is higher as an analyte concentration increases, and the color intensity of the second test portion (132) is lower as the analyte concentration increases, and wherein the color intensity of the first test portion (131) occurs based on a sandwich immunoassay scheme, and the color intensity of the second test portion (132) occurs based on a competitive immunoassay scheme;
wherein the diagnosis unit (200) is configured to:
determine**,** for a first sample, a first ratio between a color intensity of the first test portion (131) and a color intensity of the second test portion (132) at a first time-point;
determine, for a second sample, a second ratio between a color intensity of the first test portion (131) and a color intensity of the second test portion (132) at a second time-point; and
diagnose the pregnancy related state of the subject based on the first ratio and the second ratio,
wherein the second time-point is temporally subsequent to the first time-point.

2. The diagnostic device (1) of claim 1, wherein the diagnosis unit (200) is configured to output normal information or abnormal information as information about the pregnancy related state, based on the first ratio and the second ratio;

3. The diagnostic device (1) of claim 2, wherein the diagnosis unit (200) is configured to:
output the normal information when the first ratio is greater or smaller than the second ratio; or
output the abnormal information when the first ratio is smaller or greater than the second ratio.

4. The diagnostic device (1) of claim 2, wherein the diagnosis unit (200) is configured to:
output the normal information when a value of the ratio gradually decreases from the first ratio to the second ratio as a time elapses from the first time-point to the second time-point; or
output the abnormal information when a value of the ratio gradually increases from the first ratio to the second ratio as a time elapses from the first time-point to the second time-point.

5. The diagnostic device (1) of claim 1, wherein the normal information indicates normal pregnancy or normal pregnancy maintenance, and the abnormal information indicates abnormal pregnancy or miscarriage.

6. The diagnostic device (1) of claim 1, wherein the reaction unit (100) includes:
a sample pad receiving (110) the sample therein;
a conjugate pad (120);
a reaction membrane (130) having two or more test portions; and
a wicking pad (140).

7. The diagnostic device (1) of claim 1, wherein each of the first test portion (131) and/or the second test portion (132) has an individual antigen or antibody coating.

8. The diagnostic device (1) of claim 1, wherein the reaction unit (100) further includes a control portion (133).

9. The diagnostic device of claim 2, wherein the diagnosis unit is configured to:
output the normal information when the first ratio is smaller than the second ratio; or
output the abnormal information when the first ratio is greater than the second ratio.

10. The diagnostic device of claim 2, wherein the diagnosis unit is configured to:
output the normal information when a value of the ratio gradually increases from the first ratio to the second ratio as a time elapses from the first time-point to the second time-point; or
output the abnormal information when a value of the ratio gradually decreases from the first ratio to the second ratio as a time elapses from the first time-point to the second time-point.

11. The diagnostic device (1) of claim 1, wherein the normal information indicates that the subject is able to be pregnant, wherein the abnormal information indicates that the subject is not able to be pregnant or is in a menopause state.

12. The diagnostic device (1) of claim 1, wherein the diagnosis unit (200) is configured to: when the first ratio is a minimum value among a plurality of ratios including the first ratio and the second ratio, output time-point information corresponding to the first time-point as information about the pregnancy related state.

13. The diagnostic device (1) of claim 12, wherein the time-point information is time-point information about ovulation.

14. The diagnostic device (1) of claim 1, wherein the diagnosis unit (200) is configured to:
compare a plurality of ratios including the first ratio and the second ratio with each other; and
when ratios below a threshold ratio last for a predetermined period, output the abnormal information.

15. The diagnostic device (1) of claim 1, wherein the sample includes a substance selected from a group consisting of urine, blood, plasma, serum, lymph fluid, bone marrow fluid, saliva, milk, ocular fluid, semen, brain extract, spinal fluid, joint fluid, thymus fluid, ascites, amniotic fluid, cell tissue fluid, buffer solution, tap water, waste water, sewage, groundwater and combinations thereof.

16. The diagnostic device (1) of claim 1, wherein the analyte includes a hormone selected from a group consisting of follicle stimulating hormone, luteinizing hormone, estrogen, progesterone, estradiol, human chorionic gonadotropin, and combinations thereof.

17. The diagnostic device (1) of claim 6, wherein the reaction membrane includes a material selected from a group consisting of nitrocellulose, cellulose, polyethylene terephthalate (PET), polyethersulfone (PES), glass fiber, nylon and combinations thereof.

18. The diagnostic device (1) of claim 1, wherein the state of the subject is diagnosed using a method selected from a group consisting of immunochromatography, ELISA, radioimmunoassay, enzyme immunoassay, particle aggregation assay, chemiluminescence immunoassay, real-time polymerase reaction, flow cytometry, immunosensor, radial immunodiffusion, rocket immunoelectrophoresis, two- dimensional electrophoretic analysis, liquid chromatography-mass spectrometry (LC- MS), Western blot, and combinations thereof.

19. The diagnostic device (1) of claim 1, wherein the diagnosis unit (200) includes a light emitting unit (210, 222, 212) and a light receiving unit (221, 222, 223).

20. The diagnostic device (1) of claim 1, wherein the light receiving unit (221, 222, 223) measures the color intensity based on an amount of light reflected from the reaction unit (100) when the diagnosis unit (200) irradiates light on the reaction unit (100).

21. The diagnostic device (1) of claim 1, wherein the device further includes a wireless communication module.

22. A diagnostic device set (2) including the diagnostic device (1) of claim 1, and an analysis reader into which the diagnostic device (1) is inserted.

23. A diagnostic method comprising:
receiving, by a reaction unit (100), a first sample related to a first time-point and a second sample related to a second time-point;
detecting, by a diagnosis unit (200), for each of the samples, a color intensity of each of at least two test portions (131, 132) included in the reaction unit (100); and
diagnosing, by the diagnosis unit (200), a subject state, based on a ratio or a difference between a color intensity of a first test portion (131) and a color intensity of the second test portion (132),
wherein the diagnosis unit (200) is configured to diagnose a pregnancy related state of the subject, based on the ratio or the difference between the color intensity of the first test portion (131) and the color intensity of the second test portion (132), wherein the color intensity of the first test portion (131) is higher as an analyte concentration increases, and the color intensity of the second test portion (132) is lower as the analyte concentration increases, and wherein the color intensity of the first test portion (131) occurs based on a sandwich immunoassay scheme, and the color intensity of the second test portion (132) occurs based on a competitive immunoassay scheme; wherein the diagnosis unit (200) is configured to:
determine a first ratio between a color intensity of the first test portion (131) and a color intensity of the second test portion (132) at the first time-point;
determine a second ratio between a color intensity of the first test portion (131) and a color intensity of the second test portion (132) at the second time-point; and
diagnose the pregnancy related state of the subject based on the first ratio and the second ratio, and
wherein the second time-point is temporally subsequent to the first time-point.

## Patentansprüche

1. Diagnosevorrichtung (1) zum Diagnostizieren eines Zustands einer Person auf der Grundlage eines Verhältnisses oder einer Differenz zwischen einer Farbintensität eines ersten Testabschnitts (131) und einer Farbintensität eines zweiten Testabschnitts (132) in einer Reaktionseinheit (100), die eine Probe darin aufnimmt,
wobei die Vorrichtung umfasst:
dass die Reaktionseinheit (100) die Probe darin aufnimmt und zwei oder mehr Testabschnitte aufweist; und
eine Diagnoseeinheit (200) zum Diagnostizieren des Zustands der Person auf der Grundlage des Verhältnisses oder der Differenz zwischen der Farbintensität des ersten Testabschnitts (131) und der Farbintensität des zweiten Testabschnitts (132);
wobei die Diagnoseeinheit (200) so konfiguriert ist, dass sie einen schwangerschaftsbezogenen Zustand der Person auf der Grundlage des Verhältnisses oder der Differenz zwischen der Farbintensität des ersten Testabschnitts (131) und der Farbintensität des zweiten Testabschnitts (132) diagnostiziert, wobei die Farbintensität des ersten Testabschnitts (131) höher ist, wenn eine Analytkonzentration zunimmt, und die Farbintensität des zweiten Testabschnitts (132) niedriger ist, wenn die Analytkonzentration zunimmt; und
wobei die Farbintensität des ersten Testabschnitts (131) auf der Grundlage eines Sandwich-Immunoassay-Schemas erfolgt und die Farbintensität des zweiten Testabschnitts (132) auf der Grundlage eines kompetitiven Immunoassay-Schemas erfolgt;
wobei die Diagnoseeinheit (200) ferner so konfiguriert ist, dass sie:
für eine erste Probe ein erstes Verhältnis zwischen einer Farbintensität des ersten Testabschnitts (131) und einer Farbintensität des zweiten Testabschnitts (132) zu einem ersten Zeitpunkt bestimmt;
für eine zweite Probe ein zweites Verhältnis zwischen einer Farbintensität des ersten Testabschnitts (131) und einer Farbintensität des zweiten Testabschnitts (132) zu einem zweiten Zeitpunkt bestimmt; und
den schwangerschaftsbezogenen Zustand der Person auf der Grundlage des ersten Verhältnisses und des zweiten Verhältnisses diagnostiziert, wobei der zweite Zeitpunkt zeitlich nach dem ersten Zeitpunkt liegt.

2. Diagnosevorrichtung (1) nach Anspruch 1, wobei die Diagnoseeinheit (200) so konfiguriert ist, dass sie auf der Grundlage des ersten Verhältnisses und des zweiten Verhältnisses normale Informationen oder unnormale Informationen als Informationen über den schwangerschaftsbezogenen Zustand ausgibt; .

3. Diagnosevorrichtung (1) nach Anspruch 2, wobei die Diagnoseeinheit (200) so konfiguriert ist, dass sie:
die normale Information ausgibt, wenn das erste Verhältnis größer oder kleiner als das zweite Verhältnis ist; oder
die anormale Information ausgibt, wenn das erste Verhältnis kleiner oder größer als das zweite Verhältnis ist.

4. Diagnosevorrichtung (1) nach Anspruch 2, wobei die Diagnoseeinheit (200) so konfiguriert ist, dass sie:
die normale Information ausgibt, wenn ein Wert des Verhältnisses allmählich vom ersten Verhältnis zum zweiten Verhältnis abnimmt, während eine Zeit vom ersten Zeitpunkt zum zweiten Zeitpunkt verstreicht; oder
die anormale Information ausgibt, wenn ein Wert des Verhältnisses allmählich von dem ersten Verhältnis auf das zweite Verhältnis ansteigt, während eine Zeit von dem ersten Zeitpunkt zu dem zweiten Zeitpunkt verstreicht.

5. Diagnosevorrichtung (1) nach Anspruch 1, wobei die normale Information eine normale Schwangerschaft oder die Aufrechterhaltung einer normalen Schwangerschaft angibt, und die unnormale Information eine unnormale Schwangerschaft oder eine Fehlgeburt angibt.

6. Diagnosevorrichtung (1) nach Anspruch 1, wobei die Reaktionseinheit (100) umfasst:
ein Probenkissen (110), in dem die Probe aufgenommen ist;
ein konjugiertes Kissen (120);
eine Reaktionsmembran (130) mit zwei oder mehr Testabschnitten; und
ein flüssigkeitstransportierendes Kissen (140).

7. Diagnosevorrichtung (1) nach Anspruch 1, wobei jeder des ersten Testabschnitts (131) und/oder des zweiten Testabschnitts (132) jeweils eine individuelle Antigen- oder Antikörperbeschichtung aufweist.

8. Diagnosevorrichtung (1) nach Anspruch 1, wobei die Reaktionseinheit (100) ferner einen Steuerabschnitt (133) umfasst.

9. Diagnosevorrichtung nach Anspruch 2, wobei die Diagnoseeinheit so konfiguriert ist, dass sie:
die normale Information ausgibt, wenn das erste Verhältnis kleiner als das zweite Verhältnis ist; oder
die unnormale Information ausgibt, wenn das erste Verhältnis größer als das zweite Verhältnis ist.

10. Diagnosevorrichtung nach Anspruch 2, wobei die Diagnoseeinheit so konfiguriert ist, dass sie:
die normale Information ausgibt, wenn ein Wert des Verhältnisses allmählich von dem ersten Verhältnis auf das zweite Verhältnis ansteigt, während eine Zeit von dem ersten Zeitpunkt zu dem zweiten Zeitpunkt verstreicht; oder
die anormale Information ausgibt, wenn ein Wert des Verhältnisses allmählich von dem ersten Verhältnis zu dem zweiten Verhältnis abnimmt, während eine Zeit von dem ersten Zeitpunkt zu dem zweiten Zeitpunkt verstreicht.

11. Diagnosevorrichtung (1) nach Anspruch 1, wobei die normale Information angibt, dass die Person in der Lage ist, schwanger zu sein, wobei die unnormale Information angibt, dass die Person nicht in der Lage ist, schwanger zu sein oder sich im Zustand der Menopause befindet.

12. Diagnosevorrichtung (1) nach Anspruch 1, wobei die Diagnoseeinheit (200) so konfiguriert ist, dass sie, wenn das erste Verhältnis ein Minimalwert aus einer Vielzahl von Verhältnissen ist, die das erste Verhältnis und das zweite Verhältnis einschließen, Zeitpunktinformationen, die dem ersten Zeitpunkt entsprechen, als Informationen über den schwangerschaftsbezogenen Zustand ausgibt.

13. Diagnosevorrichtung (1) nach Anspruch 12, wobei die Zeitpunktinformation eine Zeitpunktinformation über den Eisprung ist.

14. Diagnosevorrichtung (1) nach Anspruch 1, wobei die Diagnoseeinheit (200) so konfiguriert ist, dass sie:
eine Vielzahl von Verhältnissen, die das erste Verhältnis und das zweite Verhältnis einschließen, miteinander vergleicht; und
wenn die Verhältnisse unterhalb eines Schwellenwertes für eine vorbestimmte Zeitspanne andauern, die anormale Information ausgibt.

15. Diagnosevorrichtung (1) nach Anspruch 1, wobei die Probe eine Substanz enthält, die aus einer Gruppe ausgewählt ist, die aus Urin, Blut, Plasma, Serum, Lymphflüssigkeit, Knochenmarkflüssigkeit, Speichel, Milch, Augenflüssigkeit, Sperma, Gehirnextrakt, Rückenmarksflüssigkeit, Gelenkflüssigkeit, Thymusflüssigkeit, Aszites, Fruchtwasser, Zellgewebeflüssigkeit, Pufferlösung, Leitungswasser, Abwasser, Klärwasser, Grundwasser und Kombinationen davon besteht.

16. Diagnosevorrichtung (1) nach Anspruch 1, wobei der Analyt ein Hormon einschließt, das aus einer Gruppe ausgewählt ist, die aus follikelstimulierendem Hormon, luteinisierendem Hormon, Östrogen, Progesteron, Östradiol, menschlichem Choriongonadotropin und Kombinationen davon besteht.

17. Diagnosevorrichtung (1) nach Anspruch 6, wobei die Reaktionsmembran ein Material enthält, das aus einer Gruppe ausgewählt ist, die aus Nitrocellulose, Cellulose, Polyethylenterephthalat (PET), Polyethersulfon (PES), Glasfaser, Nylon und Kombinationen davon besteht.

18. Diagnosevorrichtung (1) nach Anspruch 1, wobei der Zustand der Person unter Verwendung eines Verfahrens diagnostiziert wird, das aus einer Gruppe ausgewählt ist, die aus Immunochromatographie, ELISA, Radioimmunoassay, Enzymimmunoassay, Partikelaggregationsassay, Chemilumineszenz-Immunoassay, Echtzeit-Polymerase-Reaktion, Durchflusszytometrie, Immunsensor, radialer Immundiffusion, Rocket-Immunelektrophorese, zweidimensionaler elektrophoretischer Analyse, Flüssigchromatographie-Massenspektrometrie (LC-MS), Western Blot und Kombinationen davon besteht.

19. Diagnosevorrichtung (1) nach Anspruch 1, wobei die Diagnoseeinheit (200) eine Lichtemissionseinheit (210, 222, 212) und eine Lichtempfangseinheit (221, 222, 223) umfasst.

20. Diagnosevorrichtung (1) nach Anspruch 1, wobei die Lichtempfangseinheit (221, 222, 223) die Farbintensität auf der Grundlage einer Lichtmenge misst, die von der Reaktionseinheit (100) reflektiert wird, wenn die Diagnoseeinheit (200) die Reaktionseinheit (100) mit Licht bestrahlt.

21. Diagnosevorrichtung (1) nach Anspruch 1, wobei die Vorrichtung außerdem ein Modul für drahtlose Kommunikation enthält.

22. Diagnosevorrichtungssatz (2), der die Diagnosevorrichtung (1) nach Anspruch 1 und eine Analyse-Leseeinrichtung enthält, in die die Diagnosevorrichtung (1) eingesetzt ist.

23. Diagnoseverfahren, umfassend:
Empfangen einer ersten Probe, die einem ersten Zeitpunkt zugeordnet ist, und einer zweiten Probe, die einem zweiten Zeitpunkt zugeordnet ist, durch eine Reaktionseinheit (100);
Detektieren einer Farbintensität von jedem von mindestens zwei in der Reaktionseinheit (100) enthaltenen Testabschnitten (131, 132) für jede der Proben durch eine Diagnoseeinheit (200); und
Diagnostizieren eines Zustands der Person durch die Diagnoseeinheit (200), basierend auf einem Verhältnis oder einer Differenz zwischen einer Farbintensität eines ersten Testabschnitts (131) und einer Farbintensität des zweiten Testabschnitts (132),
wobei die Diagnoseeinheit (200) so konfiguriert ist, dass sie einen schwangerschaftsbezogenen Zustand der Person auf der Grundlage des Verhältnisses oder der Differenz zwischen der Farbintensität des ersten Testabschnitts (131) und der Farbintensität des zweiten Testabschnitts (132) diagnostiziert, wobei die Farbintensität des ersten Testabschnitts (131) höher ist, wenn eine Analytkonzentration zunimmt, und die Farbintensität des zweiten Testabschnitts (132) niedriger ist, wenn die Analytkonzentration zunimmt; und
wobei die Farbintensität des ersten Testabschnitts (131) auf der Grundlage eines Sandwich-Immunoassay-Schemas erfolgt und die Farbintensität des zweiten Testabschnitts (132) auf der Grundlage eines kompetitiven Immunoassay-Schemas erfolgt;
wobei die Diagnoseeinheit (200) so konfiguriert ist, dass sie:
ein erstes Verhältnis zwischen einer Farbintensität des ersten Testabschnitts (131) und einer Farbintensität des zweiten Testabschnitts (132) zu dem ersten Zeitpunkt bestimmt;
ein zweites Verhältnis zwischen einer Farbintensität des ersten Testabschnitts (131) und einer Farbintensität des zweiten Testabschnitts (132) zu dem zweiten Zeitpunkt bestimmt; und
den schwangerschaftsbezogenen Zustand der Person auf der Grundlage des ersten Verhältnisses und des zweiten Verhältnisses diagnostiziert, und wobei der zweite Zeitpunkt zeitlich nach dem ersten Zeitpunkt liegt.

## Revendications

1. Dispositif de diagnostic (1) pour diagnostiquer un état d'un sujet sur la base d'un rapport ou d'une différence entre une intensité de coloration d'une première prise d'essai (131) et une intensité de coloration d'une seconde prise d'essai (132) dans une unité de réaction (100) recevant un échantillon dans celle-ci,
dans lequel le dispositif comporte :
l'unité de réaction (100) recevant l'échantillon dans celle-ci et ayant deux prises d'essai ou plus ; et
une unité de diagnostic (200) pour diagnostiquer l'état du sujet sur la base du rapport ou de la différence entre l'intensité de coloration de la première prise d'essai (131) et l'intensité de coloration de la seconde prise d'essai (132) ;
dans lequel l'unité de diagnostic (200) est configurée pour diagnostiquer un état lié à une grossesse du sujet, sur la base du rapport ou de la différence entre l'intensité de coloration de la première prise d'essai (131) et l'intensité de coloration de la seconde prise d'essai (132), dans lequel l'intensité de coloration de la première prise d'essai (131) est plus élevée à mesure qu'une concentration d'analyte augmente, et l'intensité de coloration de la seconde prise d'essai (132) est moins élevée à mesure que la concentration d'analyte augmente, et
dans lequel l'intensité de coloration de la première prise d'essai (131) apparaît sur la base d'un schéma de dosage immunologique sandwich, et l'intensité de coloration de la seconde prise d'essai (132) apparaît sur la base d'un schéma de dosage immunologique par compétition ;
dans lequel l'unité de diagnostic (200) est configurée pour :
déterminer, pour un premier échantillon, un premier rapport entre une intensité de coloration de la première prise d'essai (131) et une intensité de coloration de la seconde prise d'essai (132) à un premier instant ;
déterminer, pour un second échantillon, un second rapport entre une intensité de coloration de la première prise d'essai (131) et une intensité de coloration de la seconde prise d'essai (132) à un second instant ; et
diagnostiquer l'état lié à une grossesse du sujet sur la base du premier rapport et du second rapport,
dans lequel le second instant est temporellement postérieur au premier instant.

2. Dispositif de diagnostic (1) selon la revendication 1, dans lequel l'unité de diagnostic (200) est configurée pour délivrer une information normale ou une information anormale sous forme d'information concernant l'état lié à une grossesse, sur la base du premier rapport et du second rapport.

3. Dispositif de diagnostic (1) selon la revendication 2, dans lequel l'unité de diagnostic (200) est configurée pour :
délivrer l'information normale lorsque le premier rapport est supérieur ou inférieur au second rapport ; ou
délivrer l'information anormale lorsque le premier rapport est inférieur ou supérieur au second rapport.

4. Dispositif de diagnostic (1) selon la revendication 2, dans lequel l'unité de diagnostic (200) est configurée pour :
délivrer l'information normale lorsqu'une valeur du rapport diminue graduellement du premier rapport au second rapport à mesure qu'un temps s'écoule du premier instant au second instant ; ou
délivrer l'information anormale lorsqu'une valeur du rapport augmente graduellement du premier rapport au second rapport à mesure qu'un temps s'écoule du premier instant au second instant.

5. Dispositif de diagnostic (1) selon la revendication 1, dans lequel l'information normale indique une grossesse normale ou une progestation normale, et l'information anormale indique une grossesse anormale ou une fausse couche.

6. Dispositif de diagnostic (1) selon la revendication 1, dans lequel l'unité de réaction (100) comporte :
un tampon d'échantillon (110) recevant l'échantillon dans celui-ci ;
un tampon de conjugué (120) ;
une membrane de réaction (130) ayant deux prises d'essai ou plus ; et
un tampon de capillarité (140).

7. Dispositif de diagnostic (1) selon la revendication 1, dans lequel chaque prise parmi la première prise d'essai (131) et/ou la seconde prise d'essai (132) a un revêtement d'antigène ou d'anticorps individuel.

8. Dispositif de diagnostic (1) selon la revendication 1, dans lequel l'unité de réaction (100) comporte en outre une prise témoin (133).

9. Dispositif de diagnostic selon la revendication 2, dans lequel l'unité de diagnostic est configurée pour :
délivrer l'information normale lorsque le premier rapport est inférieur au second rapport ; ou
délivrer l'information anormale lorsque le premier rapport est supérieur au second rapport.

10. Dispositif de diagnostic selon la revendication 2, dans lequel l'unité de diagnostic est configurée pour :
délivrer l'information normale lorsqu'une valeur du rapport augmente graduellement du premier rapport au second rapport à mesure qu'un temps s'écoule du premier instant au second instant ; ou
délivrer l'information anormale lorsqu'une valeur du rapport diminue graduellement du premier rapport au second rapport à mesure qu'un temps s'écoule du premier instant au second instant.

11. Dispositif de diagnostic (1) selon la revendication 1, dans lequel l'information normale indique que le sujet peut être enceinte, dans lequel l'information anormale indique que le sujet ne peut pas être enceinte ou est dans un état de ménopause.

12. Dispositif de diagnostic (1) selon la revendication 1, dans lequel l'unité de diagnostic (200) est configurée pour : lorsque le premier rapport est une valeur minimale parmi une pluralité de rapports comportant le premier rapport et le second rapport, délivrer une information temporelle correspondant au premier instant sous la forme d'une information concernant l'état lié à une grossesse.

13. Dispositif de diagnostic (1) selon la revendication 12, dans lequel l'information temporelle est une information temporelle concernant une ovulation.

14. Dispositif de diagnostic (1) selon la revendication 1, dans lequel l'unité de diagnostic (200) est configurée pour :
comparer une pluralité de rapports comportant le premier rapport et le second rapport les uns avec les autres ; et
lorsque des rapports inférieurs à un rapport de seuil durent pendant une période prédéterminée, délivrer l'information anormale.

15. Dispositif de diagnostic (1) selon la revendication 1, dans lequel l'échantillon comporte une substance choisie parmi un groupe constitué de : urine, sang, plasma, sérum, liquide lymphatique, liquide de moelle osseuse, salive, lait, liquide oculaire, sperme, extrait cérébral, liquide spinal, liquide articulaire, liquide de thymus, ascite, liquide amniotique, liquide de tissus cellulaires, solution tampon, eau du robinet, eau résiduaire, eaux d'égout, eau souterraine et combinaisons de ceux-ci.

16. Dispositif de diagnostic (1) selon la revendication 1, dans lequel l'analyte comporte une hormone choisie parmi un groupe constitué de : hormone de stimulation folliculaire, hormone lutéinisante, estrogène, progestérone, estradiol, gonadotropine chrorionique humaine et combinaisons de ceux-ci.

17. Dispositif de diagnostic (1) selon la revendication 6, dans lequel la membrane de réaction comporte un matériau choisi parmi un groupe constitué de : nitrocellulose, cellulose, polyéthylène téréphtalate (PET), polyéthersul-fone (PES), fibre de verre, nylon et combinaisons de ceux-ci.

18. Dispositif de diagnostic (1) selon la revendication 1, dans lequel l'état du sujet est diagnostiqué en utilisant un procédé choisi parmi un groupe constitué de : immunochromatographie, ELISA, radioimmunoanalyse, essai immuno-enzymatique, essai d'agrégation de particules, immunoanalyse par chimiluminescence, réaction d'amplification en temps réel par polymérase, cytomé-trie de flux, immunocapteur, immunodiffusion radiale, immunoélectropho-rèse en fusée, analyse électrophorétique bidimensionnelle, chromatographie en masse liquide-spectrométrie de masse (LC- MS), transfert Western et combinaisons de ceux-ci.

19. Dispositif de diagnostic (1) selon la revendication 1, dans lequel l'unité de diagnostic (200) comporte une unité d'émission de lumière (210, 222, 212) et une unité de réception de lumière (221, 222, 223).

20. Dispositif de diagnostic (1) selon la revendication 1, dans lequel l'unité de réception de lumière (221, 222, 223) mesure l'intensité de coloration sur la base d'une quantité de lumière réfléchie par l'unité de réaction (100) lorsque l'unité de diagnostic (200) projette de la lumière sur l'unité de réaction (100).

21. Dispositif de diagnostic (1) selon la revendication 1, dans lequel le dispositif comporte en outre un module de communication sans fil.

22. Ensemble de dispositif de diagnostic (2) comportant le dispositif de diagnostic (1) de la revendication 1, et un lecteur d'analyse dans lequel le dispositif de diagnostic (1) est inséré.

23. Procédé de diagnostic comprenant de :
recevoir, par une unité de réaction (100), un premier échantillon associé à un premier instant et un second échantillon associé à un second instant ;
détecter, par une unité de diagnostic (200), pour chacun des échantillons, une intensité de coloration de chaque prise parmi au moins deux prises d'essai (131, 132) comprises dans l'unité de réaction (100) ; et
diagnostiquer, par l'unité de diagnostic (200), un état de sujet, sur la base d'un rapport ou d'une différence entre une intensité de coloration d'une première prise d'essai (131) et une intensité de coloration de la seconde prise d'essai (132),
lorsque l'unité de diagnostic (200) est configurée pour diagnostiquer un état lié à une grossesse du sujet, sur la base du rapport ou de la différence entre l'intensité de coloration de la première prise d'essai (131) et l'intensité de coloration de la seconde prise d'essai (132), dans lequel l'intensité de coloration de la première prise d'essai (131) est plus élevée à mesure qu'une concentration d'analyte augmente, et l'intensité de coloration de la seconde prise d'essai (132) est moins élevée à mesure que la concentration d'analyte augmente, et
dans lequel l'intensité de coloration de la première prise d'essai (131) apparaît sur la base d'un schéma de dosage immunologique sandwich, et l'intensité de coloration de la seconde prise d'essai (132) apparaît sur la base d'un schéma de dosage immunologique par compétition ;
dans lequel l'unité de diagnostic (200) est configurée pour :
déterminer un premier rapport entre une intensité de coloration de la première prise d'essai (131) et une intensité de coloration de la seconde prise d'essai (132) au premier instant ;
déterminer un second rapport entre une intensité de coloration de la première prise d'essai (131) et une intensité de coloration de la seconde prise d'essai (132) au second instant ; et
diagnostiquer l'état lié à une grossesse du sujet sur la base du premier rapport et du second rapport, et
dans lequel le second instant est temporellement postérieur au premier instant.
